# EUROPÄISCHE PATENTSCHRIFT

(11) **EP 0 471 261 B1**
(45) Veröffentlichungstag und Bekanntmachung des Hinweises auf die Patenterteilung: **17.01.1996**
(21) Anmeldenummer: 91113091.2
(22) Anmeldetag: 03.08.1991
(51) Int. Cl.: C07D 239/42, C07D 239/32, C07D 401/04, C07D 405/10, C07D 409/04, C07D 417/04, A01N 43/54, A01N 43/78

(54) **Pyrimidyl-substituierte Acrylsäureester**
Pyrimidyl-substituted acrylic acid esters
Esters de l'acide acryliques substitués par un groupe pyrimidine

(30) Priorität: 16.08.1990 DE 4025891
(43) Veröffentlichungstag der Anmeldung: 19.02.1992
(73) Patentinhaber: BAYER AG, D-51368 Leverkusen (DE)
(72) Erfinder: Klausener, Alexander, Dr., W-4150 Krefeld 1 (DE); Knüppel, Peter C., Dr., W-5632 Wermelskirchen 3 (DE); Dehne, Heinz-Wilhelm, Dr., W-4019 Monheim (DE); Dutzmann, Stefan, Dr., W-4010 Hilden 1 (DE)

(56) Entgegenhaltungen:
- EP-A- 0 057 440
- EP-A- 0 178 826
- EP-A- 0 212 859
- US-A- 3 553 252
- US-A- 4 652 569

## Beschreibung

Die Erfindung betrifft pyrimidyl-substituierte Acrylsäureester, ein Verfahren zu ihrer Herstellung, ihre Verwendung in Schädlingsbekämpfungsmitteln und Zwischenprodukte.

Es ist bekannt, daß bestimmte substituierte Acrylsäure-ester, wie beispielsweise die Verbindung 3-Methoxy-2-(2-methylphenyl)-acrylsäuremethylester fungizide Eigenschaften besitzen (vgl. z.B. EP 178 826 und EP 212 859). Außerdem ist bekannt, daß bestimmte Pyrimidine, wie beispielsweise die Verbindung 2,4-Dichlor-5-methylthiopyrimidinyl-6-thiocyanat ebenfalls fungizide Eigenschaften besitzen (vgl. z.B. US 4.652.569: DE-OS 3509437).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden pyrimidyl-substituierte Acrylsäureester der allgemeinen Formel (I), 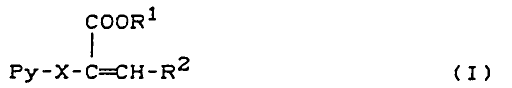
in welcher
- R¹: für geradkettiges oder verzweigtes Alkyl mit 1 bis 6 Kohlenstoffatomen steht,
- R²: für Dialkylamino mit jeweils 1 bis 6 Kohlenstoffatomen in den einzelnen Alkylteilen steht oder für einen Rest -O-R³ steht,
- X: für Sauerstoff, Schwefel oder für einen Rest 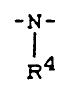 steht und
- Py: für jeweils einfach oder mehrfach, gleich oder verschieden substituiertes 2-Pyrimidyl, 4-Pyrimidyl oder 5-Pyrimidyl steht, wobei als Substituenten jeweils infrage kommen:
Halogen, Cyano, Nitro, jeweils geradkettiges oder verzweigtes Alkyl, Alkoxy oder Alkylthio mit jeweils 1 bis 9 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, jeweils gegebenenfalls einfach oder mehrfach durch C₁-C₄-Alkyl substituiertes Cycloalkyl oder Cycloalkenyl mit jeweils 3 bis 7 Kohlenstoffatomen, zweifach verknüpftes Alkandiyl mit 3 bis 5 Kohlenstoffatomen oder jeweils im Arylteil oder im Heteroarylteil gegebenenfalls einfach oder mehrfach gleich oder verschieden durch
- Halogen, Alkyl, Cycloalkyl, Alkoxy, Alkylthio, Halogenalkyl, Halogenalkoxy, Halogenalkylthio, Formyl, Dioxyalkylen, halogensubstituiertes Dioxyalkylen oder jeweils gegebenenfalls substituiertes Phenyl, Phenoxy, Benzyl, Phenylethyl, Phenylethenyl oder Phenylethinyl
substituiertes Aryl, Aryloxy, Arylthio, Arylamino, N-Alkyl-arylamino, Arylcarbonyl, Aralkyl, Arylalkenyl, Arylalkinyl, Arylalkyloxy, Arylalkylthio, Heteroaryl, Heteroaryloxy, Heteroarylthio, Heteroarylamino, N-Alkyl-heteroarylamino, Heteroarylcarbonyl, Heteroarylalkyl, Heteroarylalkenyl, Heteroarylalkinyl, Heteroarylalkyloxy oder Heteroarylalkylthio mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenyl-bzw. Alkinylteil;
wobei
- R³: für geradkettiges oder verzweigtes Alkyl mit 1 bis 6 Kohlenstoffatomen oder für Aralkyl mit 1 bis 4 Kohlenstoffatomen im geradkettigen oder verzweigten Alkylteil und 6 bis 10 Kohlenstoffatomen im Arylteil steht, und
- R⁴: für Wasserstoff, für geradkettiges oder verzweigtes Alkyl mit 1 bis 6 Kohlenstoffatomen oder für jeweils gegebenenfalls im Arylteil einfach oder mehrfach, gleich oder verschieden substituiertes Aralkyl oder Aryl mit 1 bis 6 Kohlenstoffatomen im geradkettigen oder verzweigten Alkylteil und 6 bis 10 Kohlenstoffatomen im jeweiligen Arylteil steht, wobei als Substituenten im Arylteil jeweils die bei Py genannten infrage kommen, gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen pyrimidyl-substituierten Acrylsäureester der allgemeinen Formel (I), 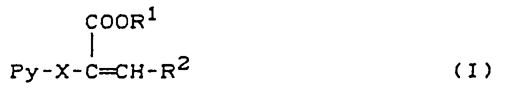
in welcher
- R¹: für Alkyl steht,
- R²: für Dialkylamino oder für einen Rest -O-R³ steht,
- X: für Sauerstoff, Schwefel oder für einen Rest 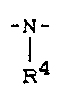 steht und
- Py: für gegebenenfalls substituiertes Pyrimidyl steht,
wobei

- R³: für Alkyl oder für gegebenenfalls substituiertes Aralkyl steht und
- R⁴: für Wasserstoff, Alkyl oder für jeweils gegebenenfalls substituiertes Aralkyl oder Aryl steht,
deren Isomere oder Isomerengemische erhält, wenn man substituierte Essigsäureester der Formel (II),

Py-X-CH₂-COOR¹ (II)

in welcher
R¹, Py und X die oben angegebene Bedeutung haben,
mit Alkoxy-bis-(dialkylamino)-methan-Verbindungen der Formel (III), 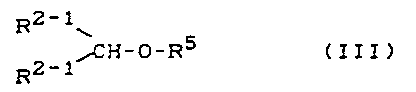
in welcher
- R²⁻¹: für Dialkylamino steht und
- R⁵: für Alkyl oder Cycloalkyl steht,
gegebenenfalls in Gegenwart eines Verdünnungsmittels umsetzt und gegebenenfalls anschließend in einer 2. Stufe die so erhältlichen 3-Dialkylaminoacrylsäurederivate der Formel (Ia), 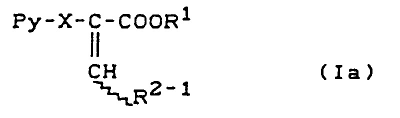
in welcher
R¹, R²⁻¹, Py und X die oben angegebene Bedeutung haben,
mit verdünnten Mineralsäuren hydrolysiert und anschließend in einer 3. Stufe die so erhältlichen 3-Hydroxyacrylsäureester der Formel (IV), 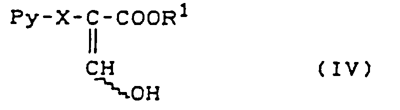
in welcher
R¹, X und Py die oben angegebene Bedeutung haben,
mit Alkylierungsmitteln der Formel (V),

R³-E¹ (V)

in welcher
- E¹: für eine elektronenanziehende Abgangsgruppe steht
und
- R³: die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen pyrimidyl-substituierten Acrylsäureester der allgemeinen Formel (I) eine gute Wirkung gegen Schädlinge besitzen,

Überraschenderweise zeigen die erfindungsgemäßen pyrimidyl-substituierten Acrylsäureester der allgemeinen Formel (I) eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester oder die Verbindung 2,4-Dichlor-5-methylthiopyrimidinyl-6-thiocyanat, welche chemisch und wirkungsmäßig naheliegende Verbindungen sind.

Die erfindungsgemäßen pyrimidyl-substituierten Acrylsäureester sind durch die Formel (I) allgemein definiert. Bevorzugt sind Verbindungen der Formel (I), bei welchen
- R¹: für Methyl, Ethyl, n- oder i-Propyl, n-, i-, s- oder t-Butyl steht,
- R²: für Dialkylamino mit jeweils 1 bis 4 Kohlenstoffatomen in den einzelnen Alkylteilen steht oder für einen Rest -O-R³ steht,
- X: für Sauerstoff, Schwefel oder für einen Rest 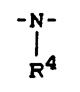 steht und
- Py: für jeweils ein- bis dreifach, gleich oder verschieden substituiertes 2-Pyrimidyl oder 4-Pyrimidyl steht, wobei mindestens ein Substituent jeweils für gegebenenfalls ein- bis dreifach, gleich oder verschieden
- durch Fluor, Chlor, Brom, Methyl, Ethyl, n-oder i-Propyl, n-, i-, s- oder t-Butyl, Methoxy, Ethoxy, n- oder i-Propoxy, Methylthio, Ethylthio, Trifluormethyl, Trifluormethoxy, Formyl, Trifluormethylthio, Dioxymethylen, Dioxyethylen, Tetrafluordioxyethylen, Difluordioxymethylen, Cyclopentyl, Cyclohexyl oder durch jeweils gegebenenfalls ein- bis dreifach, gleich oder verschieden [durch Fluor, Chlor, Methyl, Methoxy oder Trifluormethyl] substituiertes Phenyl, Phenoxy, Benzyl, Phenylethyl, Phenylethenyl oder Phenylethinyl -
substituiertes Phenyl, Naphthyl, Phenoxy, Phenylthio, N-Methyl-phenylamino, Phenylcarbonyl, Benzyl, Phenylethyl, Phenylpropyl, Phenylethenyl, Phenylethinyl, Benzyloxy, Cyclohexenyl oder Heteroaryl steht, wobei als Heteroarylreste im einzelnen die folgenden infrage kommen: 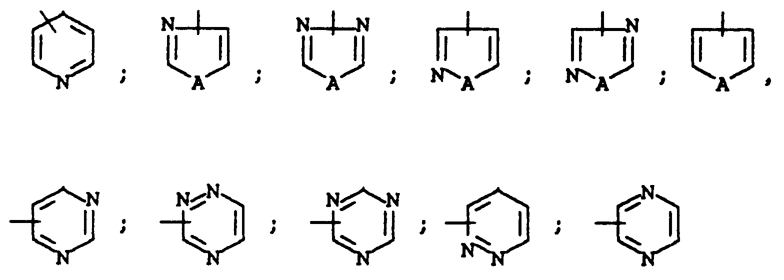 welche gegebenenfalls auch benzanelliert sein können und bei welchen

- A: jeweils für Sauerstoff, Schwefel oder eine NH-Gruppe steht;
darüberhinaus kommen als weitere Pyrimidylsubstituenten vorzugsweise die folgenden infrage: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trichlormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Dimethylcarbamoyl, Diethylcarbamoyl, Allyl, Butenyl, Ethinyl, Vinyl, Propargyl, Cyclopentyl, Cyclohexyl, 1,3-Propandiyl oder 1,4-Butandiyl;
wobei
- R³: für Methyl, Ethyl, n- oder i-Propyl, n-, i-, s- oder t-Butyl oder für Benzyl steht und
- R⁴: für Wasserstoff, Methyl, Ethyl, n- oder i-Propyl, n-, i-, s- oder t-Butyl oder für Benzyl oder Phenyl steht.

Ganz besonders bevorzugt sind Verbindungen der Formel (I), bei welchen
- R¹: für Methyl oder Ethyl steht,
- R²: für Methoxy oder Ethoxy steht,
- X: für einen Rest 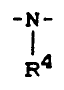 steht und
- Py: für jeweils ein- bis dreifach, gleich oder verschieden substituiertes 2-Pyrimidyl oder 4-Pyrimidyl steht, wobei mindestens ein Substituent jeweils für gegebenenfalls ein- bis dreifach, gleich oder verschieden durch Fluor, Chlor, Brom, Methyl, Ethyl, t-Butyl, Methoxy, Ethoxy, Trifluormethyl, Trifluormethoxy, Dioxymethylen, Difluordioxymethylen, Dioxyethylen, Tetrafluordioxyethylen, Phenylethenyl, Phenylethinyl, Benzyl, Cyclohexyl, Phenoxy, Methoxyphenyl, Formyl oder Phenyl substituiertes und/oder benzanelliertes Phenyl, Naphthyl, Phenoxy, Cyclohexenyl, Cyclohexyl, Benzyl, Pyridyl, Pyrimidyl, Pyrazolyl, Oxazolyl, Thiazolyl, Thienyl, Furyl, Thiadiazolyl, Oxadiazolyl, Imidazolyl oder Triazolyl steht
und als zusätzliche Pyrimidylsubstituenten jeweils infrage kommen: Fluor, Chlor, Brom, Cyano, Nitro, Methyl, Ethyl, Methoxy, Ethoxy, Methylthio, Trifluormethyl, Trichlormethyl, Trifluormethoxy, Trifluormethylthio, Methoxycarbonyl, Ethoxycarbonyl Dimethylamino, Cyclopentyl, Cyclohexyl, 1,3-Propandiyl oder 1,4-Butandiyl und wobei
- R⁴: für Methyl, Ethyl oder Benzyl steht.

Aryl als solches oder in Zusammensetzungen bedeutet Phenyl oder Naphthyl, insbesondere Phenyl.

Alle aliphatischen Reste als solche oder in Zusammensetzungen sind geradkettig oder verzweigt.

Halogen steht, wenn nicht anders definiert, für Fluor, Chlor, Brom oder Iod, insbesondere für Fluor, Chlor oder Brom.

Im einzelnen seien außer den bei den Herstellungsbeispielen genannten Verbindungen die folgenden pyrimidylsubstituierten Acrylsäureester der allgemeinen Formel (I) genannt:

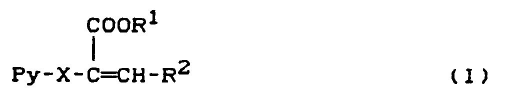

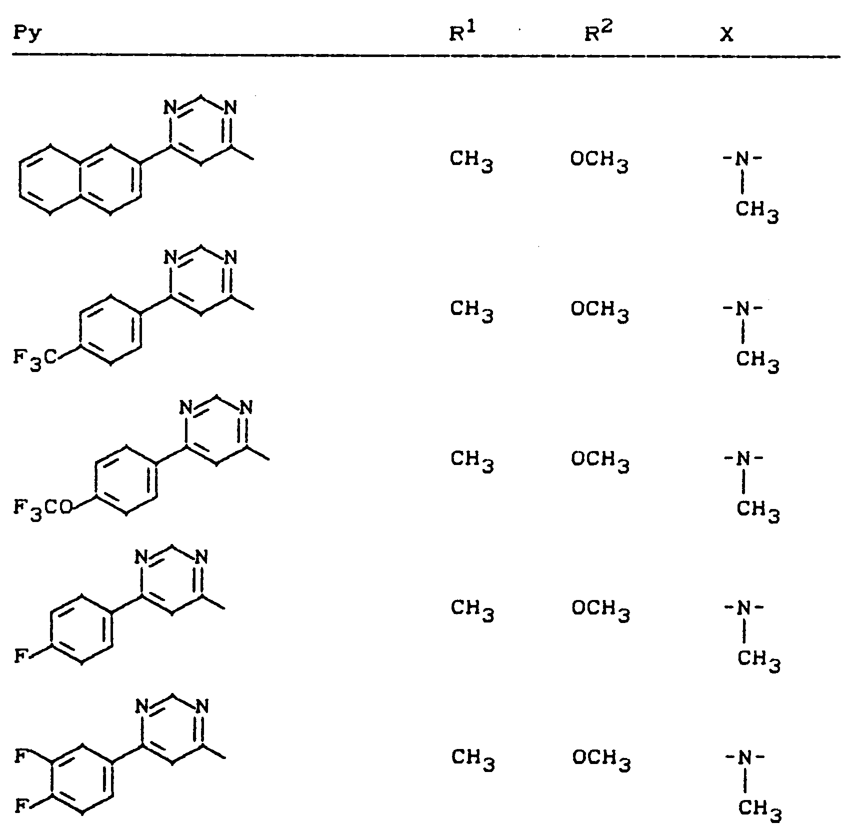

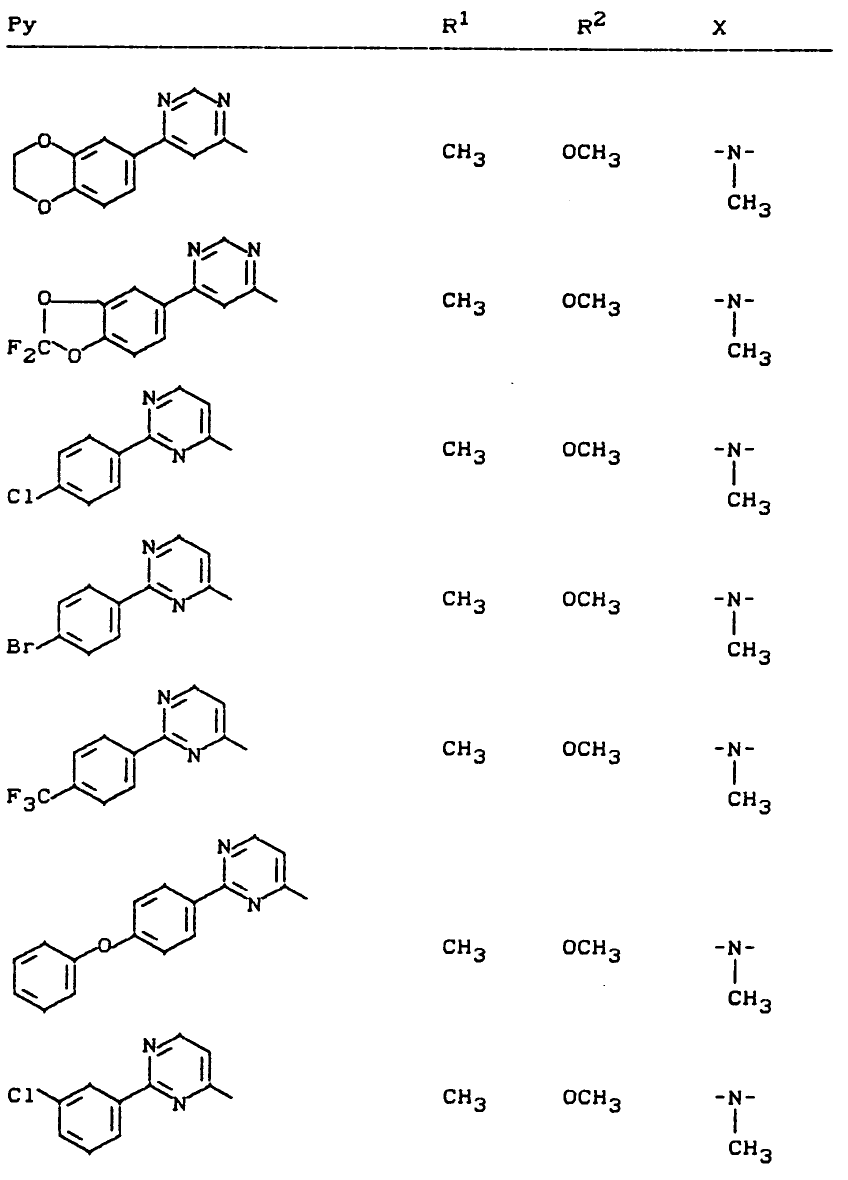

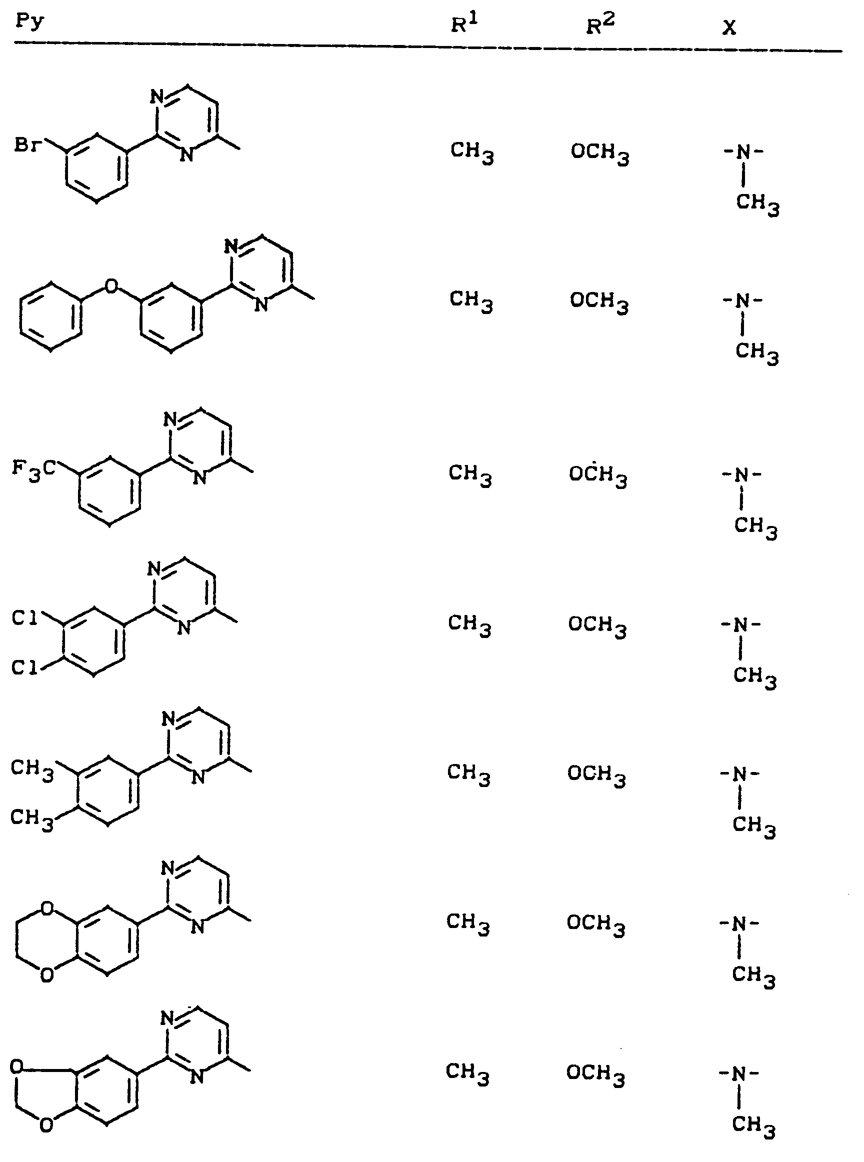

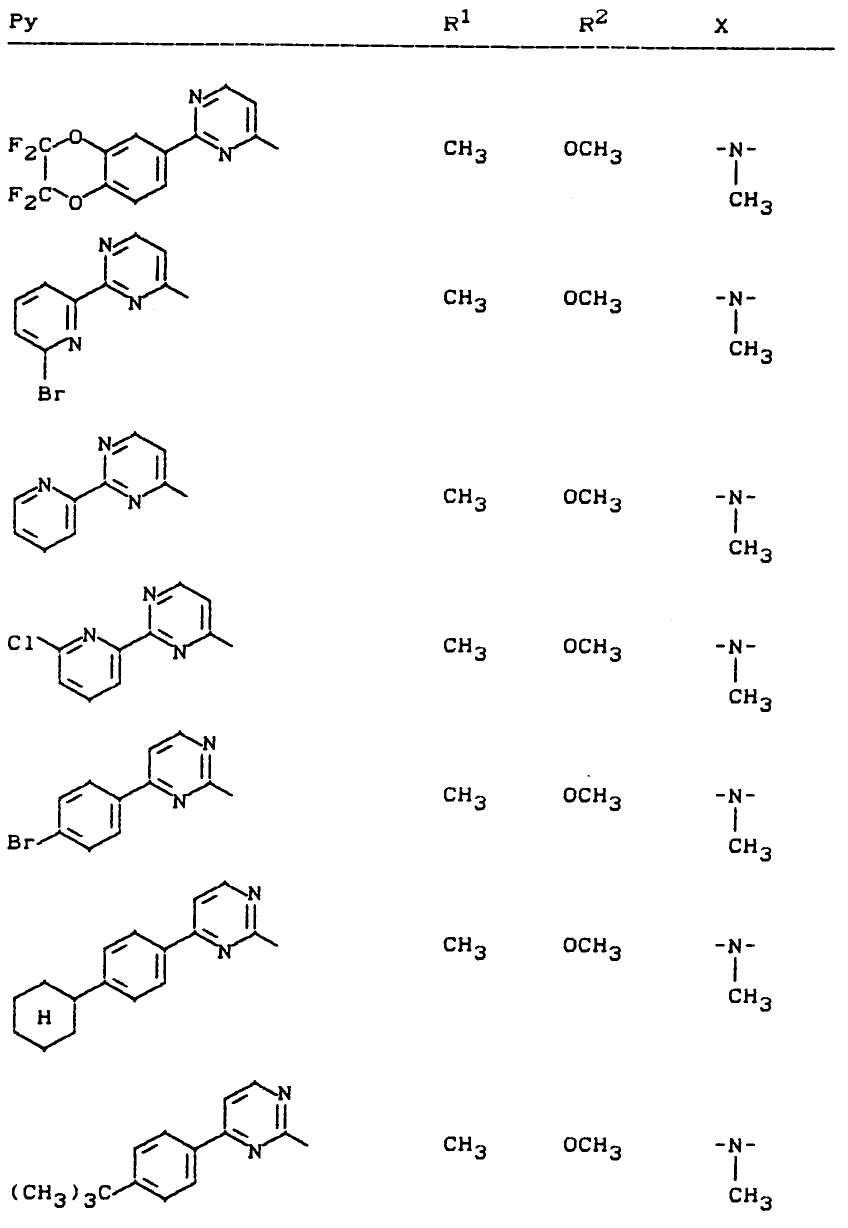

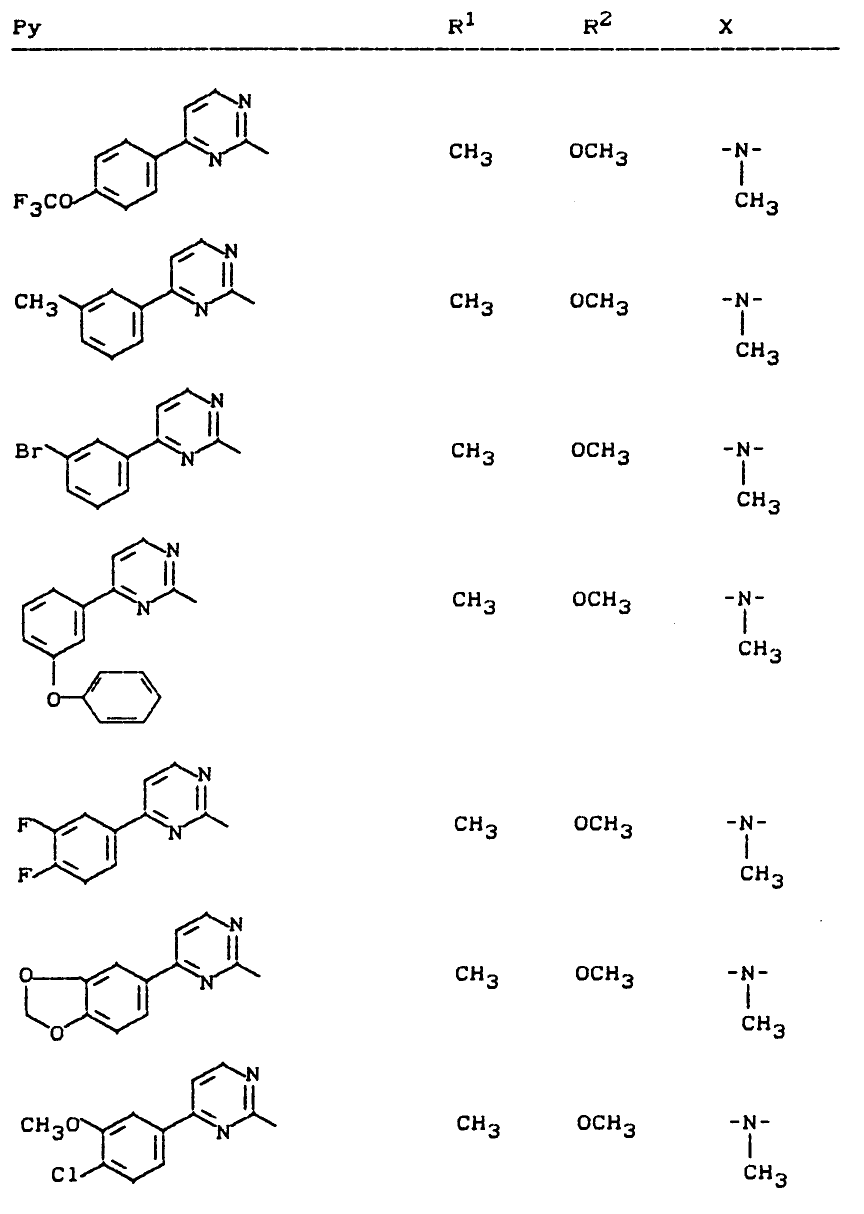

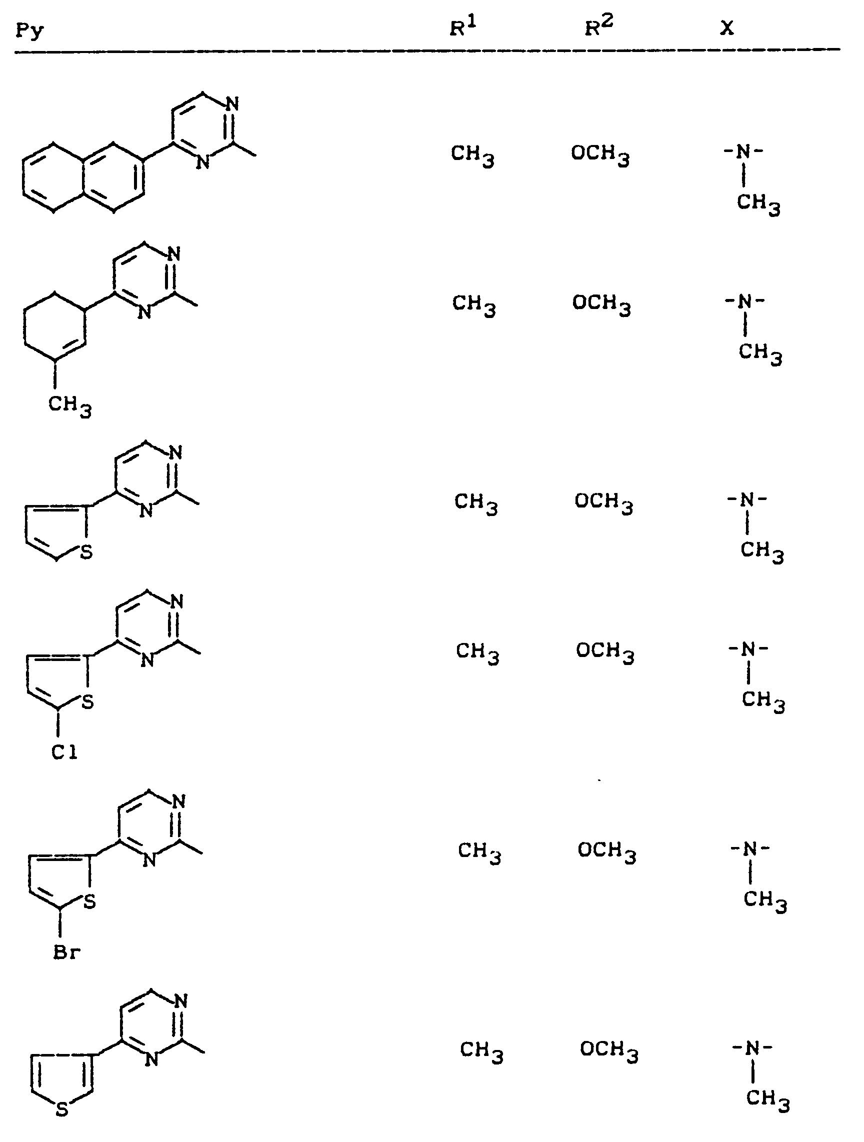

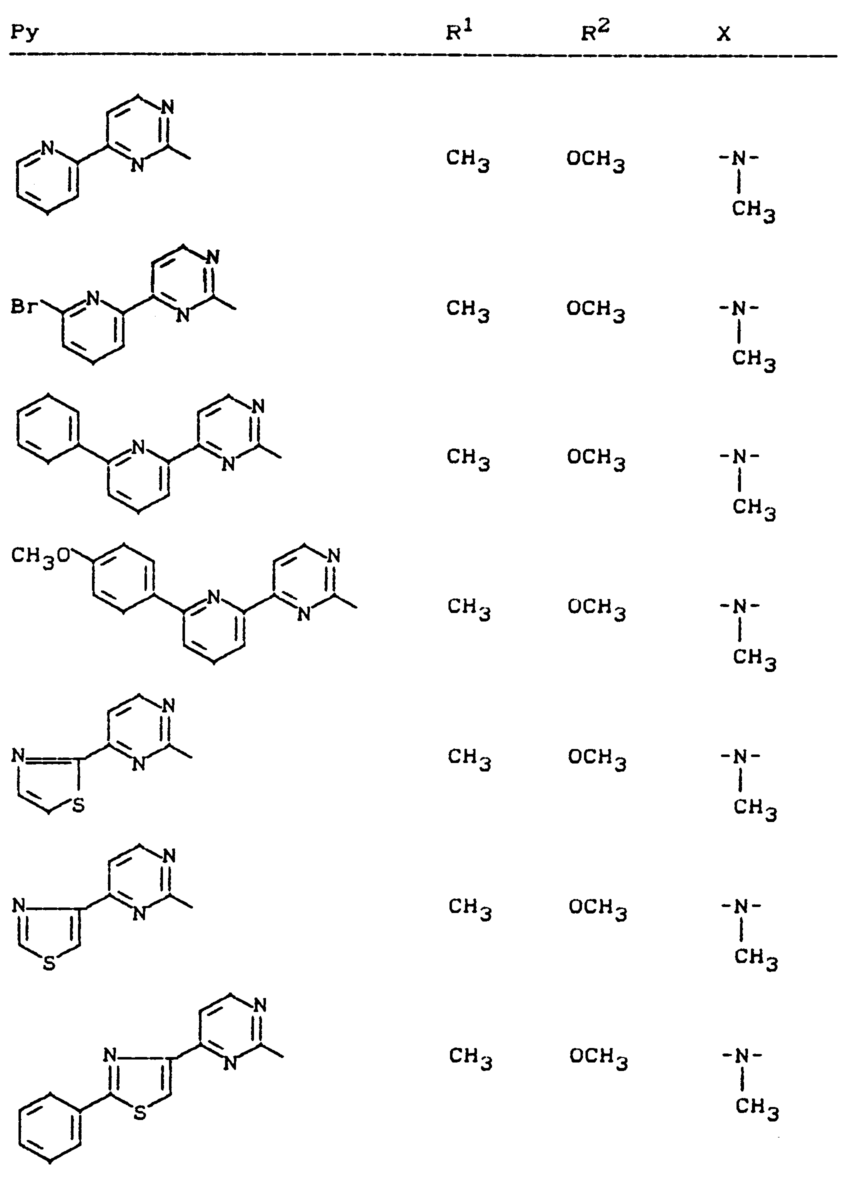

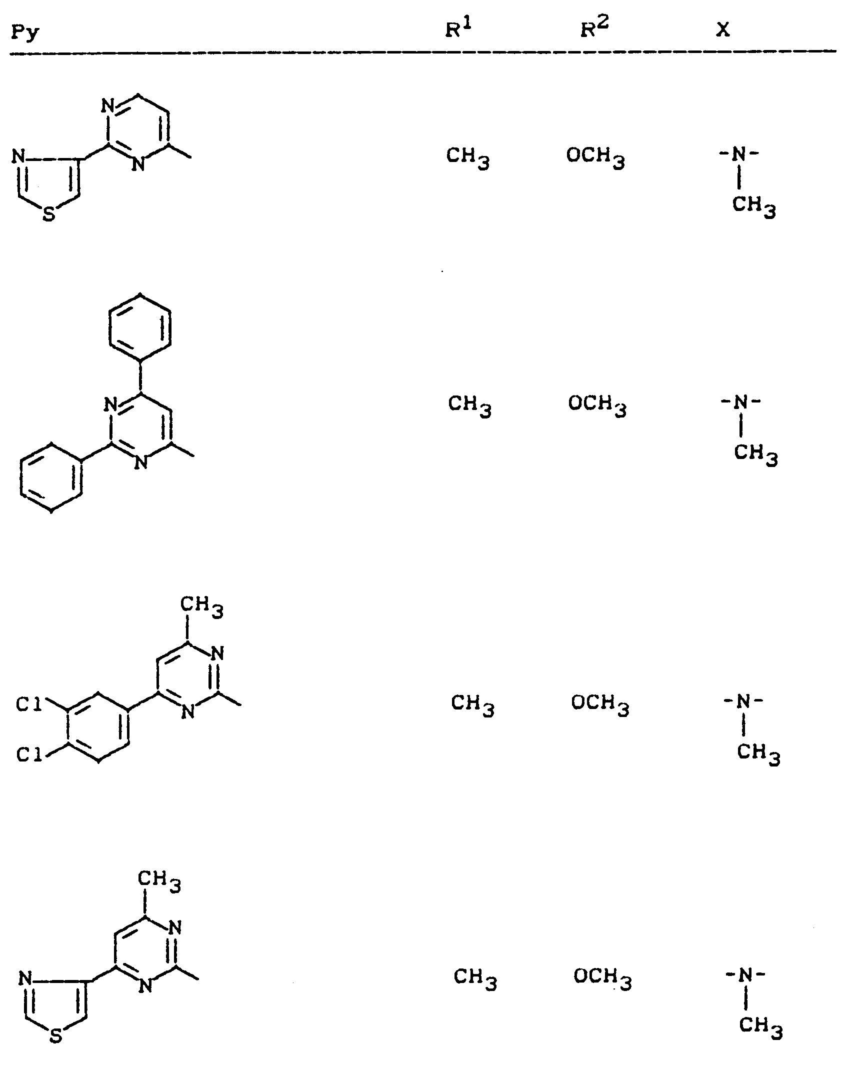

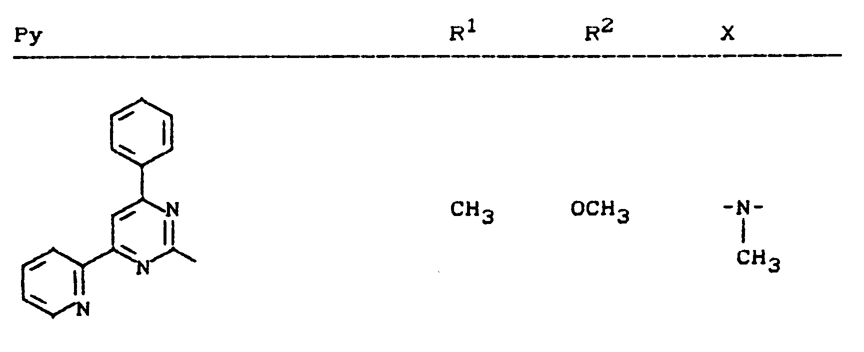

Verwendet man beispielsweise N-[4-(6-Phenyl)-pyrimidinyl]-N-methylaminoessigsäuremethylester und t-Butoxy- bis (dimethylamino)-methan sowie Dimethylsulfat als Ausgangsstoffe, so läßt sich der Reaktionsablauf des erfindungsgemäßen Verfahrens durch das folgende Formelschema darstellen: 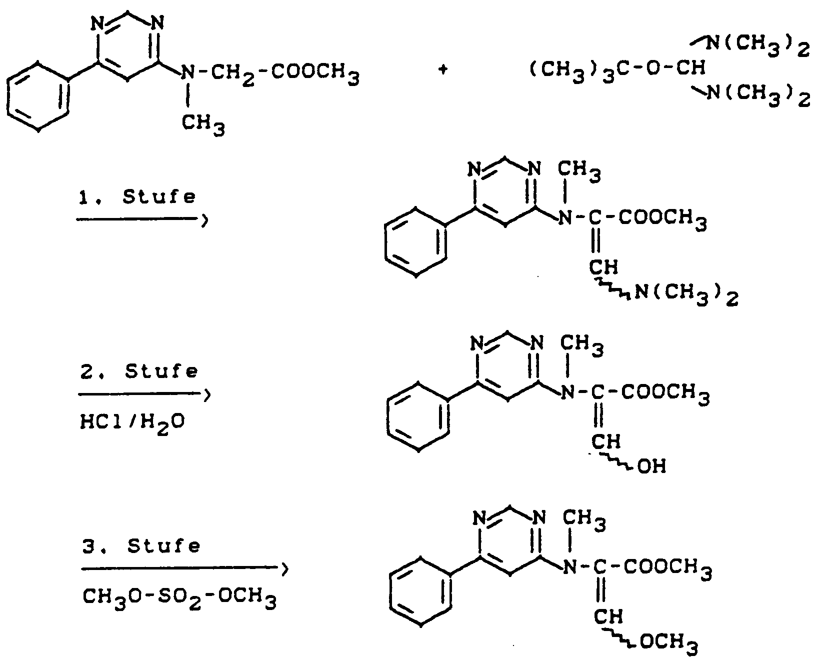
Die zur Durchführung des erfindungsgemäßen Verfahrens als Ausgangsstoffe benötigten substituierten Essigsäureester sind durch die Formel (II) allgemein definiert. In dieser Formel (II) stehen R¹, Py und X vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (II) sind teilweise bekannt (vgl. z.B. Liebigs Ann. Chem. 1988, 633-642; Anal. Chem. 58, 1681-1685 [1986]; JP 58198472; ZA 7308543; DE-OS 2342881) oder erhältlich in Analogie zu bekannten Verfahren (vgl. z.B. Chem Pharm. Bull. 32, 497-503 [1984]; EP 326389; Austral. J. Chem. 32, 669-679 [1979]; EP 310550; J. Heterocycl. Chem. 18, 183-184 [1981]; J. Heterocycl. Chem. 19, 1165 [1982]; DE-OS 3807532; Rev. Trav. Chim. Pays-Bas 86, 15-25 [1967]; J. Amer. Chem. Soc. 90, 5518 ff. [1968]).

Die zur Durchführung des erfindungsgemäßen Verfahrens weiterhin als Ausgangsstoffe benötigten Alkoxy-bis-(dialkylamino)-methan-Verbindungen sind durch die Formel (III) allgemein definiert. In dieser Formel (III) steht R²⁻¹ vorzugsweise für Dialkylamino mit jeweils 1 bis 6 Kohlenstoffatomen, besonders bevorzugt 1 bis 4 Kohlenstoffatomen in den einzelnen geradkettigen oder verzweigten Alkylteilen.

R⁵ steht vorzugsweise für sekundäres oder tertiäres Alkyl mit 3 bis 8 Kohlenstoffatomen oder für Cycloalkyl mit 5 bis 7 Kohlenstoffatomen, insbesondere für t-Butyl oder Cyclohexyl.

Die Alkoxy-bis(dialkylamino)-methan-Derivate der Formel (III) sind ebenfalls bekannt (vgl. z.B. Chem. Ber. 101, 41-50 [1968]; Chem. Ber, 101, 1885-1888 [1968]; DE 2303919; PCT Int. Appl. WO 8601204) oder erhältlich in Analogie zu bekannten Verfahren. Die Zwischenprodukte der Formel IV sind neu und ebenfalls Gegenstand der Erfindung.

Die zur Durchführung des erfindungsgemäßen Verfahrens in der 3, Stufe weiterhin als Ausgangsstoffe benötigten Alkylierungsmittel sind durch die Formel (V) allgemein definiert. In dieser Formel (V) steht R³ vorzugsweise für diejenigen Reste, die bereits im Zusammenhang mit der Beschreibung der erfindungsgemäßen Stoffe der Formel (I) als bevorzugt für diesen Substituenten genannt wurden.
- E¹: steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (V) sind allgemein bekannte Verbindungen der organischen Chemie.

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl oder -diethylether, Amide wie Dimethylformamid, Dimethylacetamid, N-Methylformanilid, N-Methylpyrrolidon oder Hexamethylphosphorsäuretriamid.

Es ist auch möglich, die 1. Stufe des erfindungsgemäßen Verfahrens ganz ohne Zusatz von Lösungsmitteln durchzuführen.

Die Reaktionstemperaturen können bei der Durchführung der 1. Stufe des erfindungsgemäßen Verfahrens in einem größeren Bereich variiert werden` Im allgemeinen arbeitet man bei Temperaturen zwischen -35°C und +150°C, vorzugsweise bei Temperaturen zwischen 0°C und 120°C.

Die 1. Stufe das erfindungsgemäßen Verfahrens kann auch unter vermindertem oder erhöhtem Druck durchgeführt werden, bevorzugt arbeitet man jedoch unter Normaldruck.

Gegebenenfalls kann der Einsatz einer Inertgasatmosphäre wie beispielsweise Stickstoff oder Argon zweckmäßig sein, im allgemeinen ist es jedoch möglich, die 1. Stufe des erfindungsgemäßen Verfahrens unter normaler Raumluftatmosphäre durchzuführen.

Zur Durchführung der 1. Stufe des erfindungsgemäßen Verfahrens setzt man pro Mol an substituiertem Essigsäure ester der Formel (II) im allgemeinen 1.0 bis 15.0 Mol, vorzugsweise 1.0 bis 5.0 Mol an Alkoxy-bis-(dialkylamino)-methan-Verbindung der Formel (III) ein.

Die Reaktionsdurchführung, Aufarbeitung und Isolierung der Reaktionsprodukte erfolgt nach allgemein üblichen Methoden (vgl. auch die Herstellungsbeispiele).

Als Säuren zur Durchführung der Hydrolyse in der 2. Stufe des erfindungsgemäßen Verfahrens kommen übliche anorganische und organische Säuren infrage. Vorzugsweise verwendet man wäßrige Lösungen von anorganischen Mineralsäuren, wie Salzsäure, Schwefelsäure, Phosphorsäure oder Salpetersäure; insbesondere wäßrige Salzsäure.

Als Verdünnungsmittel zur Durchführung der 2. und 3. Stufe des erfindungsgemäßen Verfahrens kommen ebenfalls inerte organische Lösungsmittel infrage. Hierzu gehören insbesondere aliphatische, alicyclische oder aromatische, gegebenenfalls haloganierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die 2. Stufe des erfindungsgemäßen Verfahrens wird insbesondere in polaren Verdünnungsmitteln wie Acetonitril, Aceton oder Dimethylformamid gegebenenfalls auch in Gegenwart von Wasser durchgeführt.

Die 3. Stufe des erfindungsgemäßen Verfahrens kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyldimethyl-ammoniummethylsulfat, Dimethyl-C₁₂/C₁₄-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die 3. Stufe des erfindungsgemäßen Verfahrens wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. und 3. Stufe des erfindungsgemäßen Verfahrens in einem größeren Bereich variiert werden. Im allgemeinen arbeitet man bei Temperaturen zwischen -30°C und +150°C, vorzugsweise bei Temperaturen zwischen -20°C und +120°C.

Sowohl die 2. Stufe des erfindungsgemäßen Verfahrens als auch die 3. Stufe des erfindungsgemäßen Verfahrens werden im allgemeinen unter Normaldruck durchgeführt. Es ist jedoch auch möglich sie unter vermindertem oder erhöhtem Druck durchzuführen.

Zur Durchführung der 2. Stufe des erfindungsgemäßen Verfahrens setzt man pro Mol an 3-Dialkylaminoacrylsäureester der Formel (Ia) im allgemeinen 1.0 bis 3.0 Mol, vorzugsweise 1.0 bis 2.0 Mol an verdünnter Mineralsäure ein.
Die Reaktionsdurchführung, Aufarbeitung und Isolierung der Reaktionsprodukte erfolgt nach allgemein üblichen Methoden.

Zur Durchführung der 3. Stufe des erfindungsgemäßen Verfahrens setzt man pro Mol an 3-Hydroxyacrylsäureester der Formel (IV) im allgemeinen 1.0 bis 5.0 Mol, vorzugsweise 1.0 bis 3.0 Mol an Alkylierungsmittel der Formel (V) und gegebenenfalls 1.0 bis 5.0 Mol, vorzugsweise 1.0 bis 3.0 Mol an Reaktionshilfsmittel ein.
Die Reaktionsdurchführung, Aufarbeitung und Isolierung der Reaktionsprodukte erfolgt nach allgemein üblichen Methoden.

In einer besonders bevorzugten Variante führt man die 2. und die 3. Reaktionsstufe des erfindungsgemäßen Verfahrens in einer sogenannten "Eintopfreaktion" ohne Isolierung der Zwischenprodukte der Formel (IV) direkt in einem Reaktionsschritt durch. Auch bei dieser Variante erfolgt Reaktionsdurchführung, Aufarbeitung und Isolierung der Reaktionsprodukte nach allgemein üblichen Methoden (vgl. auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus an Weizen oder Gerste (Erysiphe graminis) oder gegen den Erreger der Braunfleckenkrankheit der Gerste (Cochliobolus sativus) oder gegen den Erreger des Getreideschneeschimmels (Fusarium nivale) oder gegen den Erreger der Braunspelzigkeit des Weizens (Leptosphaeria nodorum) oder gegen den Erreger der Netzfleckenkrankheit der Gerste (Pyrenophora teres) oder zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) oder zur Bekämpfung von Krankheiten im Obst- und Gemüseanbau, wie beispielsweise gegen den Erreger der Tomatenbraunfäule (Phytophthora infectans) oder gegen den Erreger des falschen Rebenmehltaus (Plasmopara viticola), einsetzen. Hervorzuheben ist auch die gute in vitro-Wirksamkeit der erfindungsgemäßen Wirkstoffe. Auch die Zwischenprodukte der Formeln (II) und (IV) sind fungizid wirksam.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

### Herstellungsbeispiele:

### Beispiel 1:

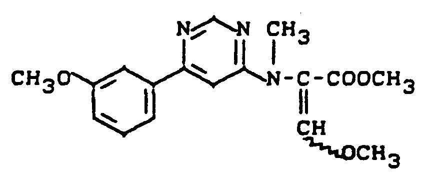

Zu 6 g (0.017 Mol) 2-{N-[6-(3-Methoxyphenyl)-pyrimidin-4-yl]-N-methylamino}-3-dimethylaminoacrylsäuremethylester in 600 ml Aceton gibt man zunächst 600 ml Wasser und anschließend 18 ml (0.035 Mol) 2normale Salzsäure und rührt anschließend 8 Stunden bei Raumtemperatur. Im Anschluß daran wird Aceton abdestilliert, die wäßrige Lösung neutralisiert und mit Essigester extrahiert. Die organische Phase wird eingeengt, der Rückstand in 100 ml Dimethylformamid aufgenommen, mit 4.8 g (0.035 Mol) Kaliumcarbonat und 4.4 g (0.035 Mol) Dimethylsulfat versetzt und 16 Stunden bei Raumtemperatur gerührt. Zur Aufarbeitung wird im Vakuum eingeengt, der Rückstand zwischen Essigester und Wasser verteilt. Die organische Phase eingeengt und der Rückstand an Kieselgel chromatographiert (Laufmittel: Essigester).

Man erhält 2.8 g (50% der Theorie) an 2-{N-[6-(3-Methoxyphenyl)-pyrimidin-4-yl]-N-methylamino}-3-methoxy-acrylsäuremethylester vom Schmelzpunkt 87°-90°C.

### Beispiel 2:

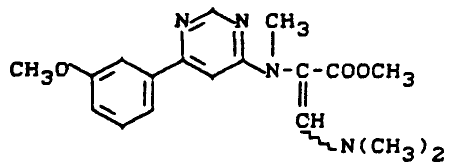

Zu 15 g (0.052 Mol) N-[6-(3-Methoxyphenyl)-4-pyrimidinyl]-N-methylglycinmethylester werden 54.6 g (0.313 Mol) t-Butyloxy-bis-(dimethylamino)methan gegeben. Nach 16-stündigem Rühren bei 80°C wird das Reaktionsgemisch auf Wasser gegeben und mit Essigester extrahiert. Die vereinigten, getrockneten Essigesterphasen werden eingeengt.

Man erhält 15.9 g (89% der Theorie) an 2-{N-[6-(3-Methoxyphenyl)pyrimidin-4-yl]-N-methylamino}-3-dimethylaminoacrylsäuremethylester vom Schmelzpunkt 172-174°C.

### Herstellung der Ausgangsverbindung:

### Beispiel II-1:

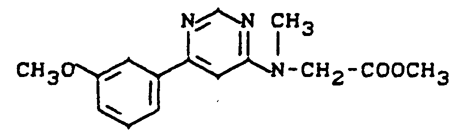

Zu 47.6 g (0.216 Mol) 4-Chlor-6-(3-methoxyphenyl)-pyrimidin in 600 ml Dioxan werden 60.2 g (0.431 Mol) Sarcosinsäuremethylesterhydrochlorid und 65.5 g (0.647 Mol) Triethylamin gegeben und dieses Reaktionsgemisch 16 Stunden auf Rückflußtemperatur erwärmt. Danach werden weitere 12.4 g (0.089 Mol) Sarcosinsäuremethylesterhydrochlorid und 13.0 g (0.128 Mol) Triethylamin zugesetzt und weitere 6 Stunden auf Rückflußtemperatur erwärmt. Zur Aufarbeitung wird mit Wasser versetzt und mit Essigester extrahiert. Die vereinigten, getrockneten Essigesterphasen werden im Vakuum eingeengt.

Man erhält 50.6 g (82% der Theorie)an N-[(3-Methoxyphenyl-4-pyrimidyl)-N-methyl]-glycinmethylester vom Schmelzpunkt 50-52°C.

### Beispiel 3:

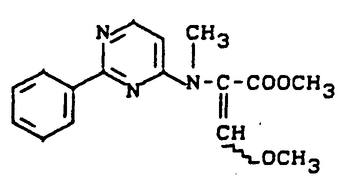

Zu 10 g (0.032 Mol) 2-[N-(2-Phenylpyrimidin-4-yl)-N-methylamino]-3-dimethylaminoacrylsäuremethylester in 100 ml Dimethylformamid gibt man 33 ml (0.064 Mol) 2normale Salzsäure, rührt 4 Stunden bei 50°C, gießt dann die Reaktionsmischung in Wasser, neutralisiert und extrahiert mit Essigester. Die Essigesterphase wird eingeengt, der Rückstand in 100 ml Dimethylformamid gelöst, mit 13.3 g (0.096 Mol) Kaliumcarbonat und 4.5 g (0.035 Mol) Dimethylsulfat versetzt und 5 Stunden bei Raumtemperatur gerührt, Zur Aufarbeitung gießt man die Reaktionsmischung in Wasser, extrahiert mit Essigester, engt die organische Phase ein und kristallisiert den Rückstand durch Verrühren mit Diisopropylether.

Man erhält 5.9 g (62% der Theorie) an 3-Methoxy-2-[N-(2-phenylpyrimidin-4-yl)-N-methylamino)-acrylsäuremethylester vom Schmelzpunkt 152-154°C.

### Beispiel 4:

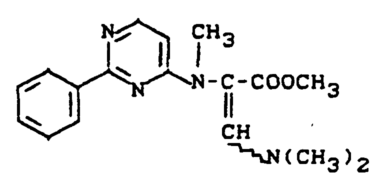

Zu 19.0 g (0.074 Mol) N-(2-Phenyl-4-pyrimidyl)-N-methylglycinmethylester werden 51.6 g (0.295 Mol) t-Butyloxy- bis-(dimethylamino)methan gegeben. Nach 16 Stunden Rühren bei 100°C wird das Reaktionsgemisch in Wasser gegossen und mit Essigester extrahiert, Die vereinigten, getrockneten Essigesterphasen werden eingeengt.

Man erhält 23.0 g (100% der Theorie) an 2-[N-(2-Phenylpyrimidin-4-yl)-N-methylamino]-3-dimethylaminoacrylsäuremethylester als Öl.
¹H-NMR (CDCl₃ Tetramethylsilan):
- δ =: 2.91 ppm (6H) N(CH₃)₂; 3.62 ppm (3H) COOCH₃;
3.34 ppm (3H) N-CH₃

### Herstellung der Ausgangsverbindung:

### Beispiel II-2:

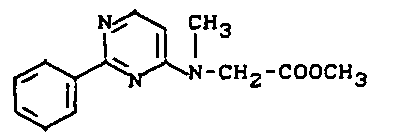

Zu 17.7 g (0.093 Mol) 4-Chlor-2-phenyl-pyrimidin in 250 ml Dioxan werden 19.5 g (0.139 Mol) Sarcosinsäuremethylesterhydrochlorid und 28.2 g (0.279 Mol) Triethylamin gegeben und dieses Reaktionsgemisch wird 20 Stunden auf 100°C erwärmt. Zur Aufarbeitung wird das Reaktionsgemisch mit Wasser versetzt und mit Essigester extrahiert, Die vereinigten, getrockneten Essigesterphasen werden im Vakuum eingeengt.

Man erhält 21.9 g (92% der Theorie) an 4-Chlor-6-(3-methoxyphenyl)-pyrimidin vom Schmelzpunkt 112-115°C.

### Beispiel 5:

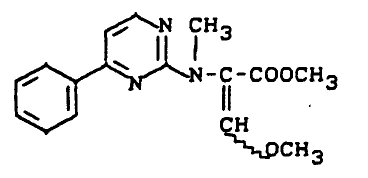

Zu 10 g (0.032 Mol) 2-[N-(4-Phenylpyrimidin-2-yl)-N-methylamino]-3-dimethylaminoacrylsäuremethylester in 80 ml Dimethylformamid gibt man 33.5 ml (0.064 Mol) 2normale Salzsäure, rührt 4 Stunden bei 50°C, gießt dann die Reaktionsmischung in Wasser, neutralisiert und extrahiert mit Essigester. Die Essigesterphase wird eingeengt, der Rückstand in 80 ml Dimethylformamid gelöst, mit 13.3 g (0.096 Mol) Kaliumcarbonat und 4.5 g (0.035 Mol) Dimethylsulfat versetzt und 16 Stunden bei Raumtemperatur gerührt, Zur Aufarbeitung gießt man die Reaktionsmischung in Wasser, extrahiert mit Essigester, engt die organische Phase ein, verrührt den Rückstand mit Diisopropylether, saugt ab und trocknet.

Man erhält 7.5 g (78% der Theorie) an 3-Methoxy-2-[N-(4-phenylpyrimidin-2-yl)-N-methylamino]-acrylsäuremethylester vom Schmelzpunkt 123°-125°C.

### Beispiel 6:

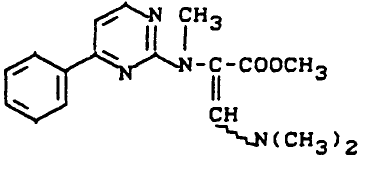

Zu 23.9 g (0.093 Mol) N-(4-Phenyl-2-pyrimidyl)-N-methylglycinmethylester werden 64.6 g (0.372 Mol) t-Butyloxy- bis-(dimethylamino)methan gegeben. Nach 20 Stunden Rühren bei 100°C wird das Reaktionsgemisch auf Wasser gegeben und mit Essigester extrahiert. Die vereinigten, getrockneten Essigesterphasen werden im Vakuum eingeengt.

Man erhält 27.0 g (93% der Theorie) an 2-[N-(4-Phenylpyrimidin-2-yl)-N-methylamino]-3-dimethylaminoacrylsäuremethylester vom Schmelzpunkt 101°C.

### Herstellung der Ausgangsverbindung:

### Beispiel II-3:

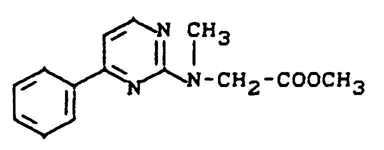

Zu 46.5 g (0.294 Mol) 2-Chlor-4-phenyl-pyrimidin in 500 ml Dioxan werden 51.1 g (0.366 Mol) Sarcosinsäuremethylesterhydrochlorid und 74.0 g (0.732 Mol) Triethylamin gegeben und dieses Reaktionsgemisch wird 16 Stunden auf 100°C erwärmt. Zur Aufarbeitung wird das Reaktionsgemisch mit Wasser versetzt und mit Essigester extrahiert. Die vereinigten, getrockneten Essigesterphasen werden im Vakuum eingeengt.

Man erhält 60.9 g (97% der Theorie) an N-(4-Phenyl-2-pyrimidyl)-N-methylglycinmethylester als Öl.
¹H-NMR (CDCl₃ Tetramethylsilan):
- δ =: 3.35 ppm (3H) N-CH₃; 4.45 ppm (2H) -CH₂-;
3.74 ppm (3H) -COOCH₃.

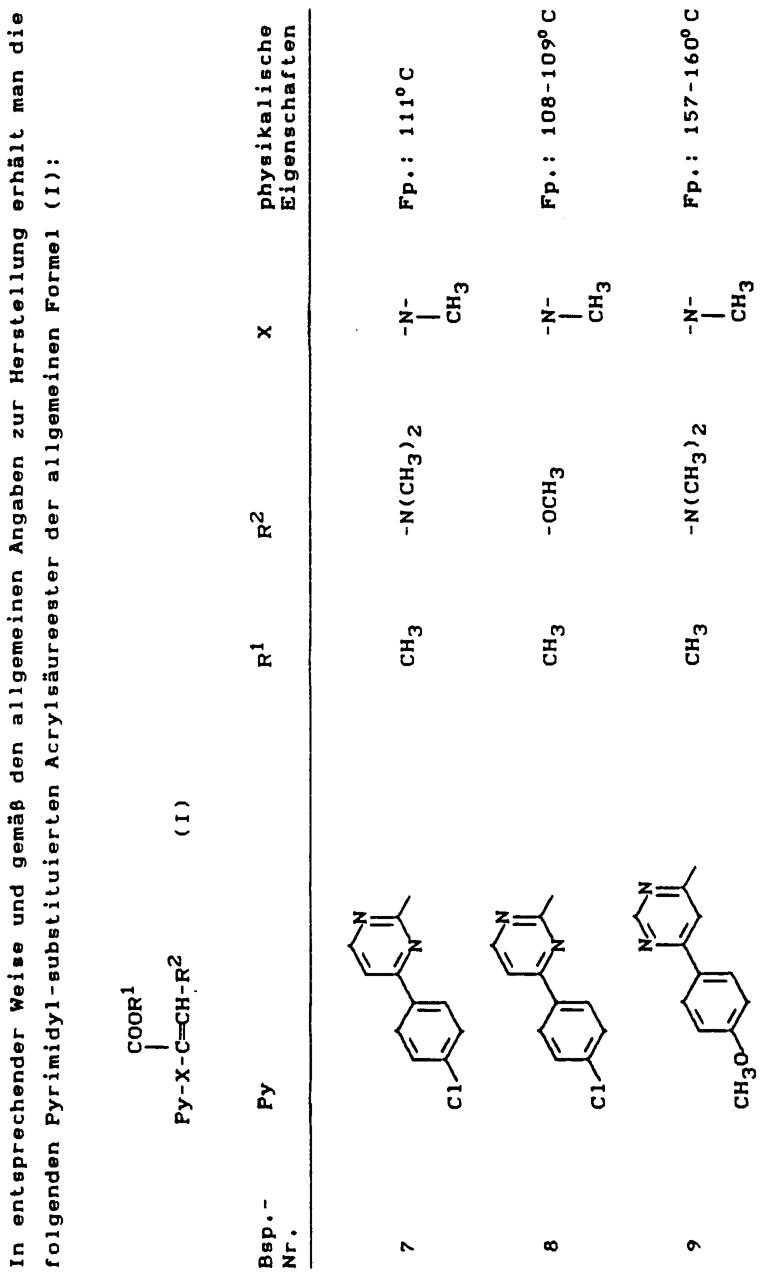

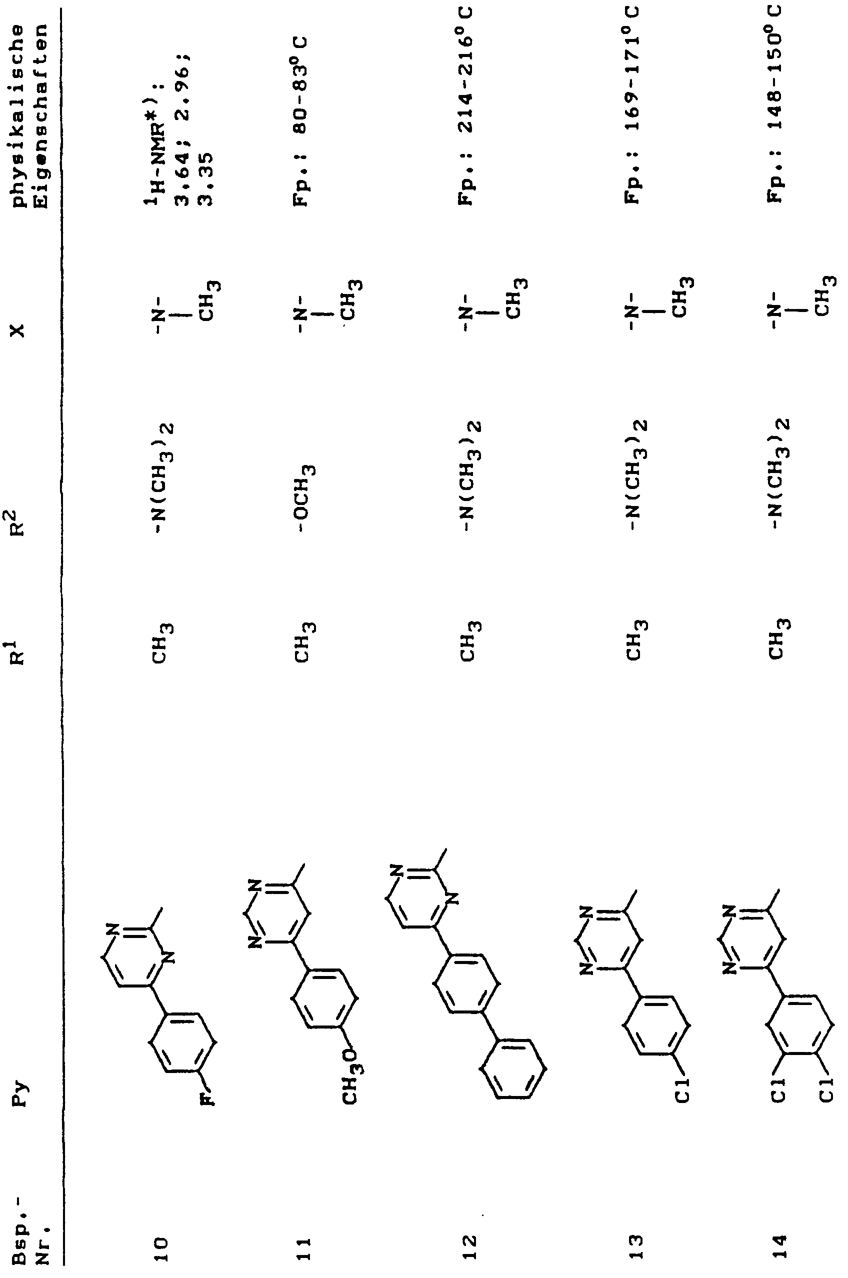

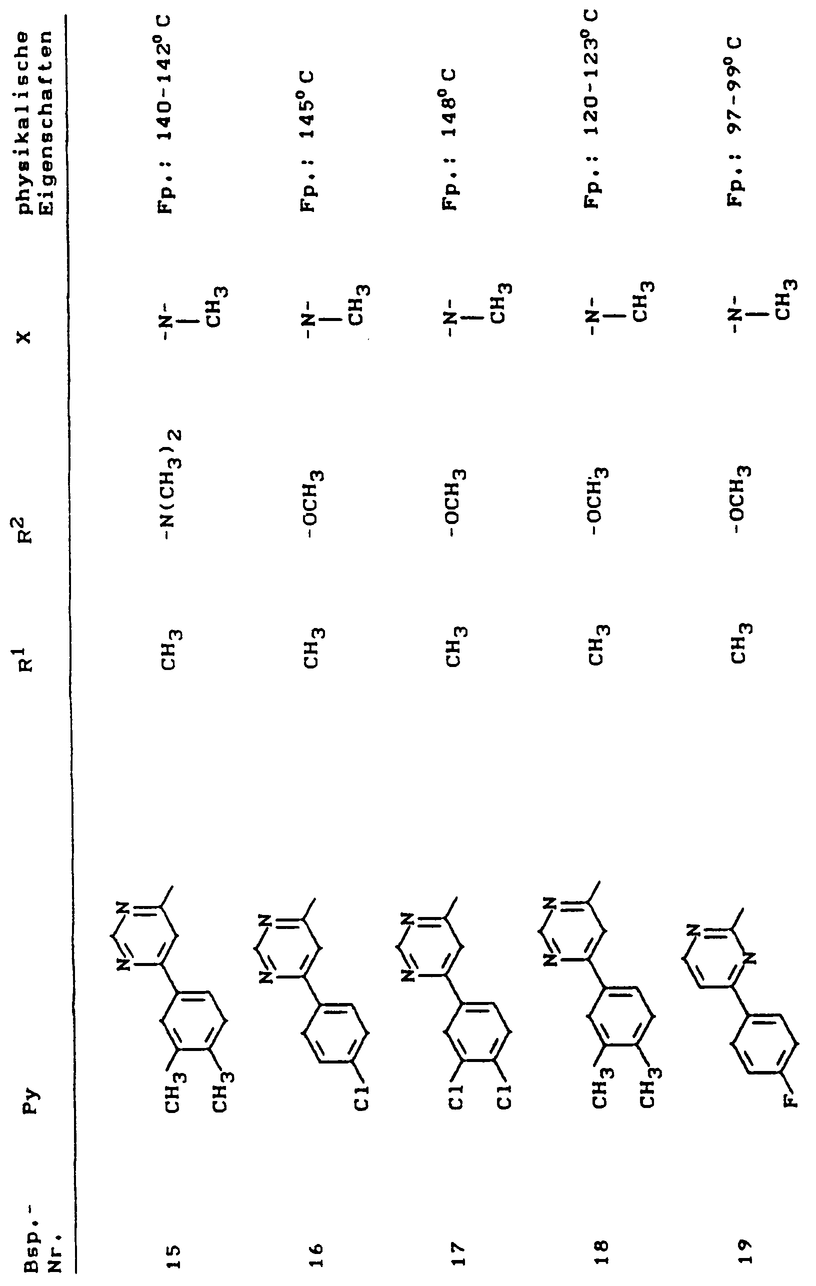

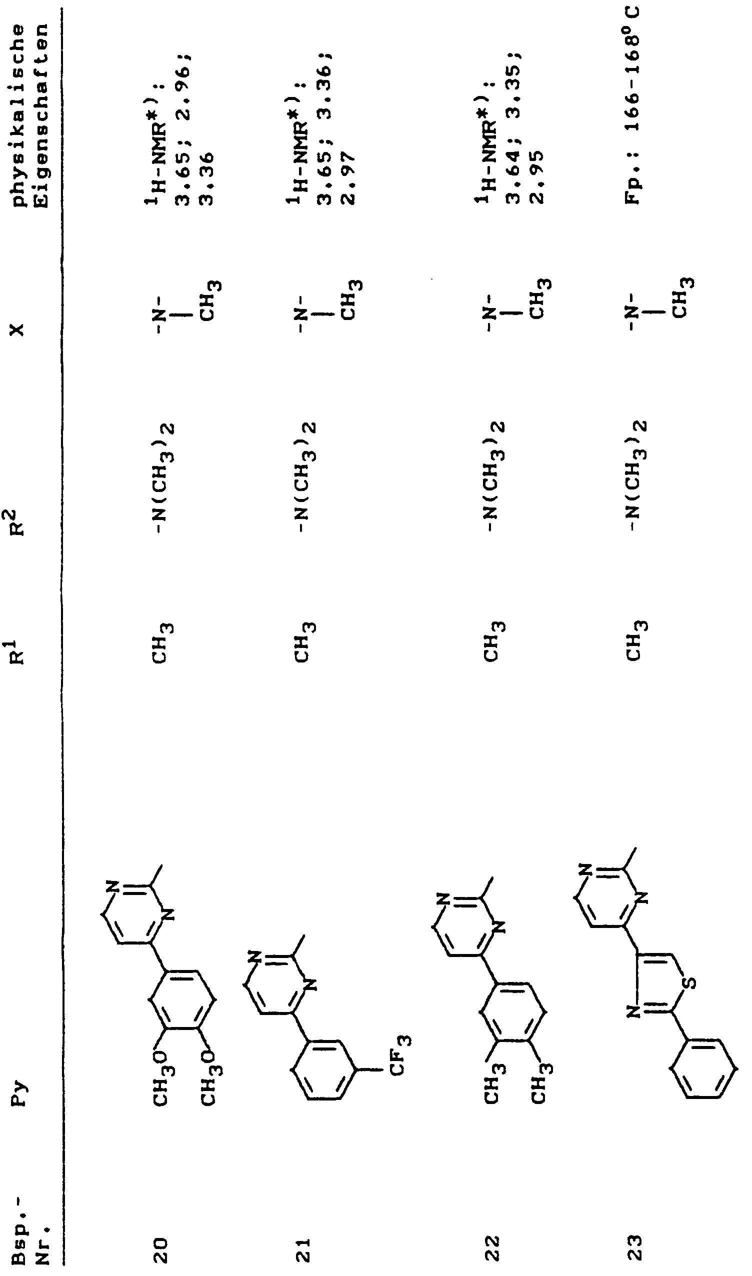

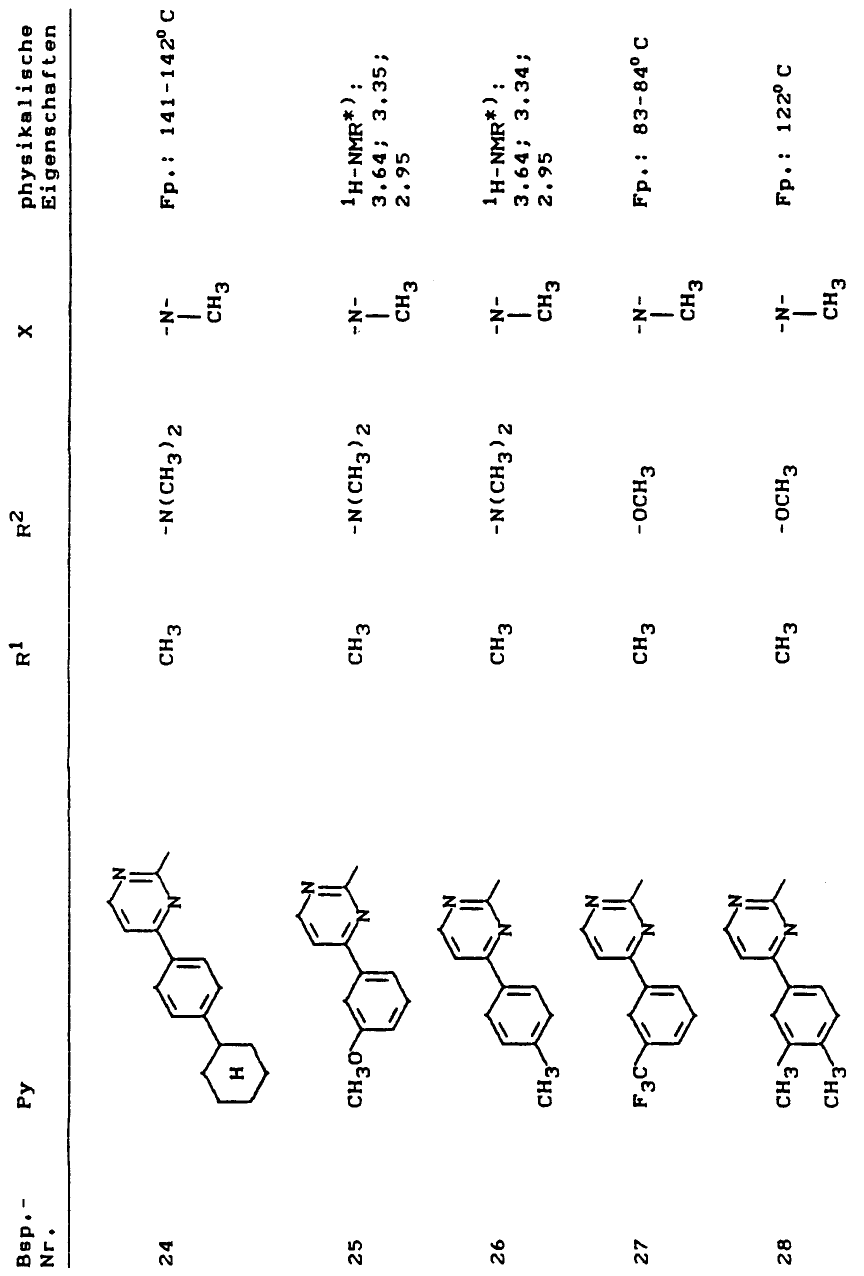

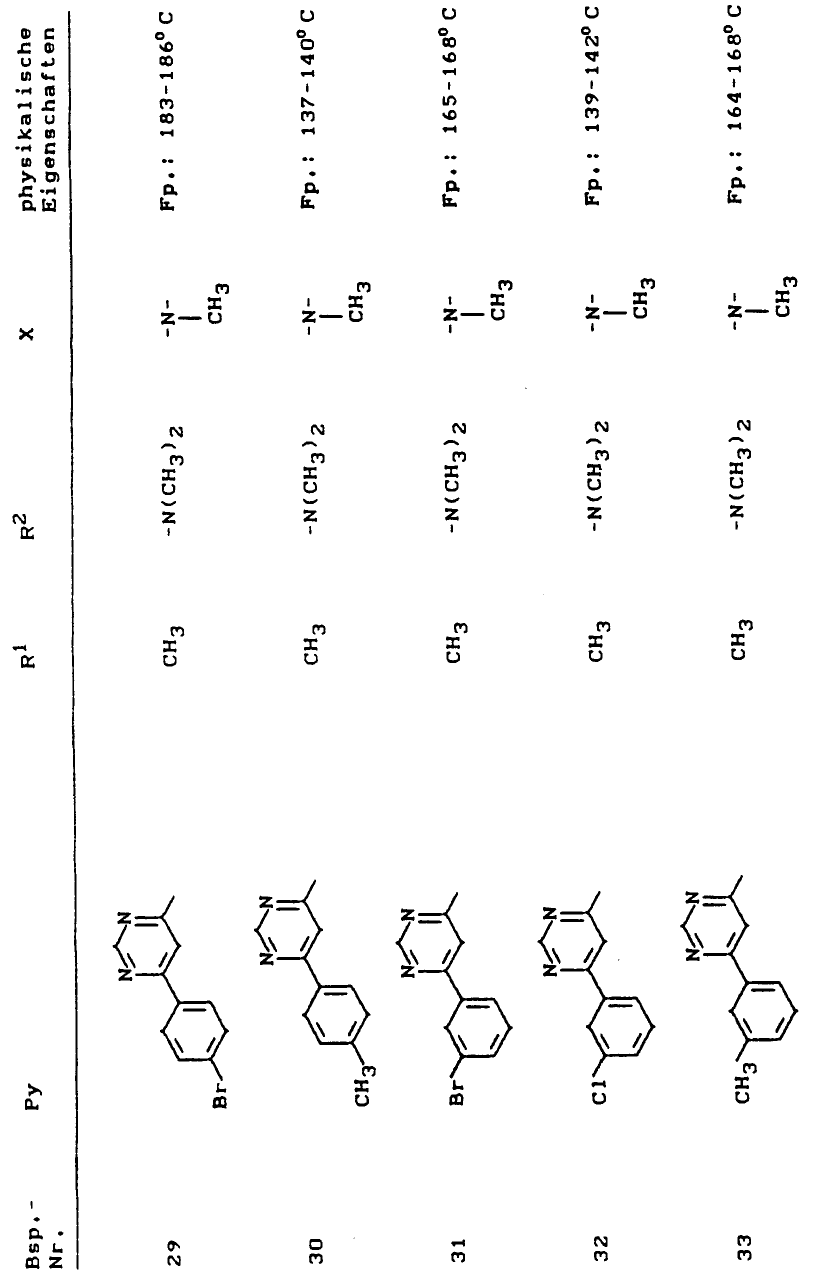

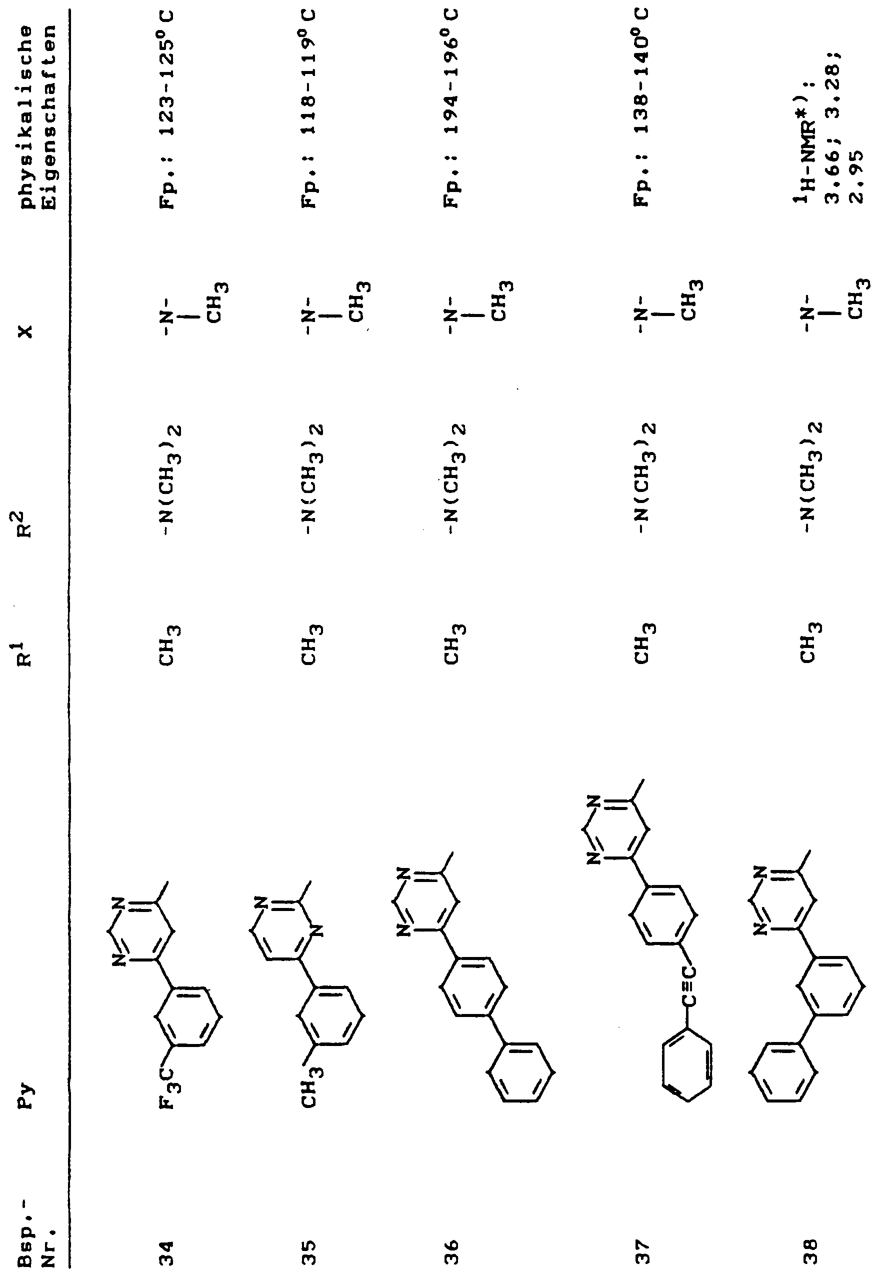

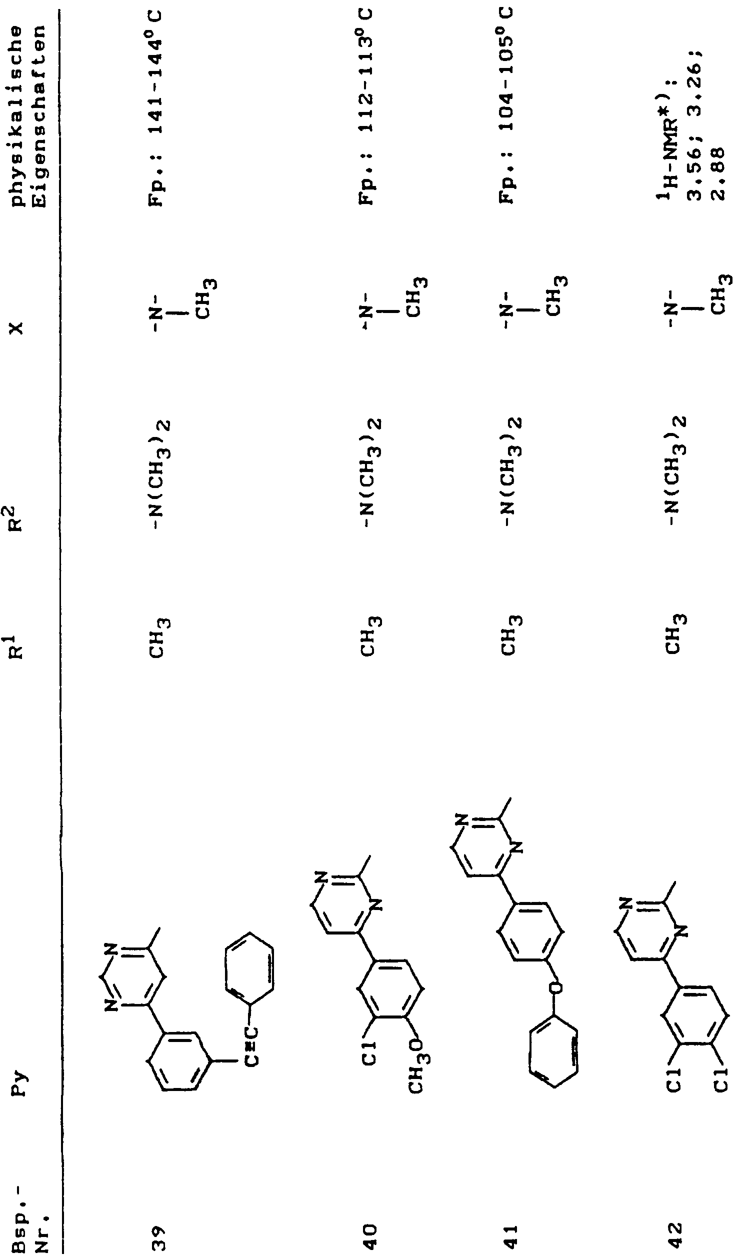

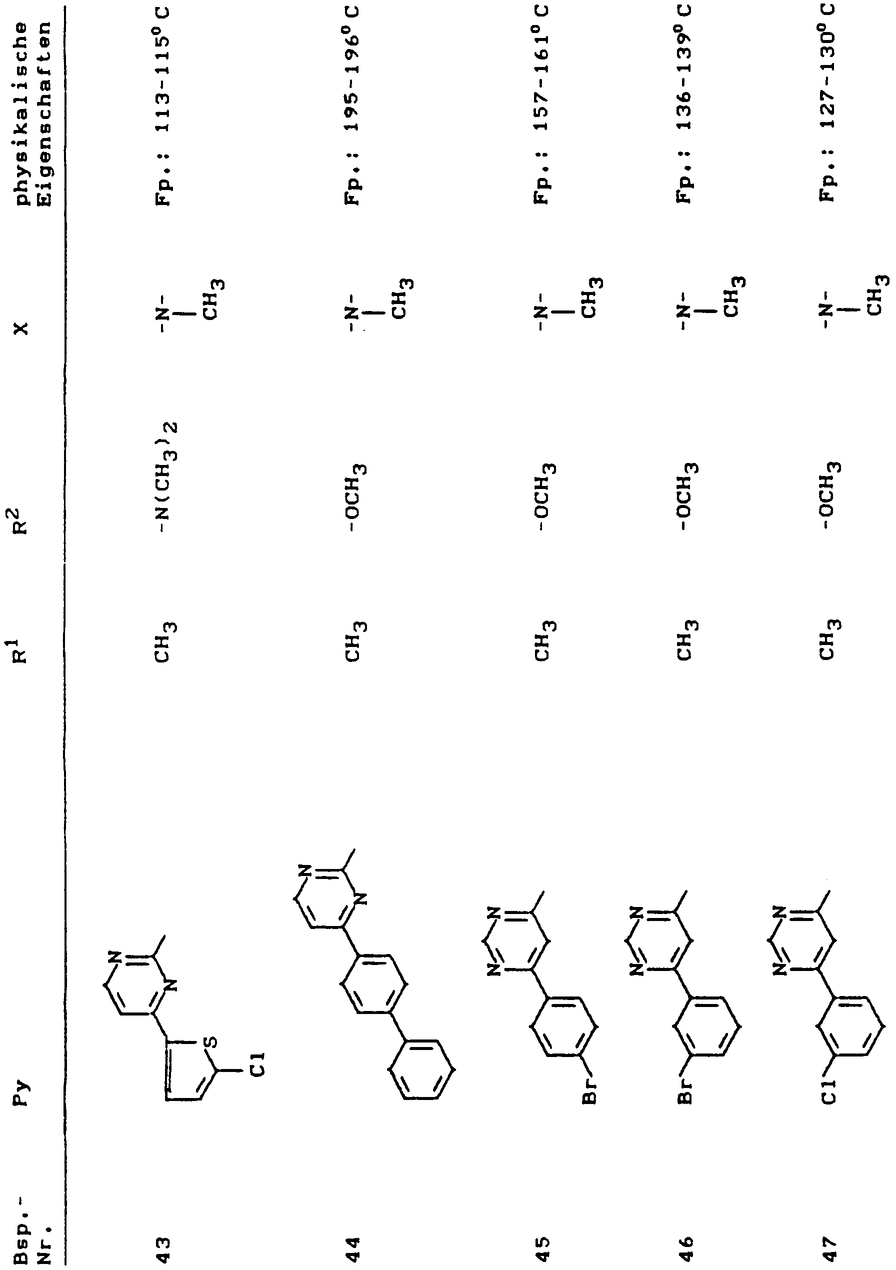

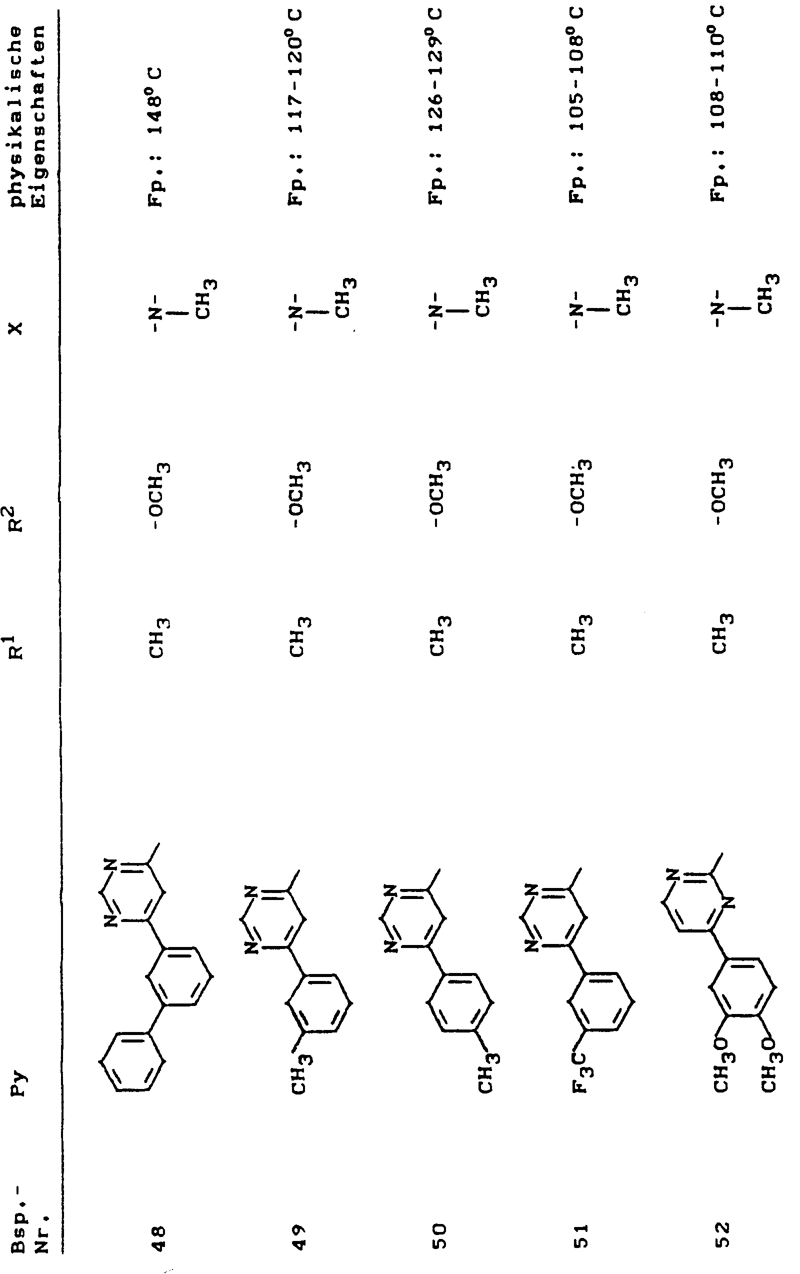

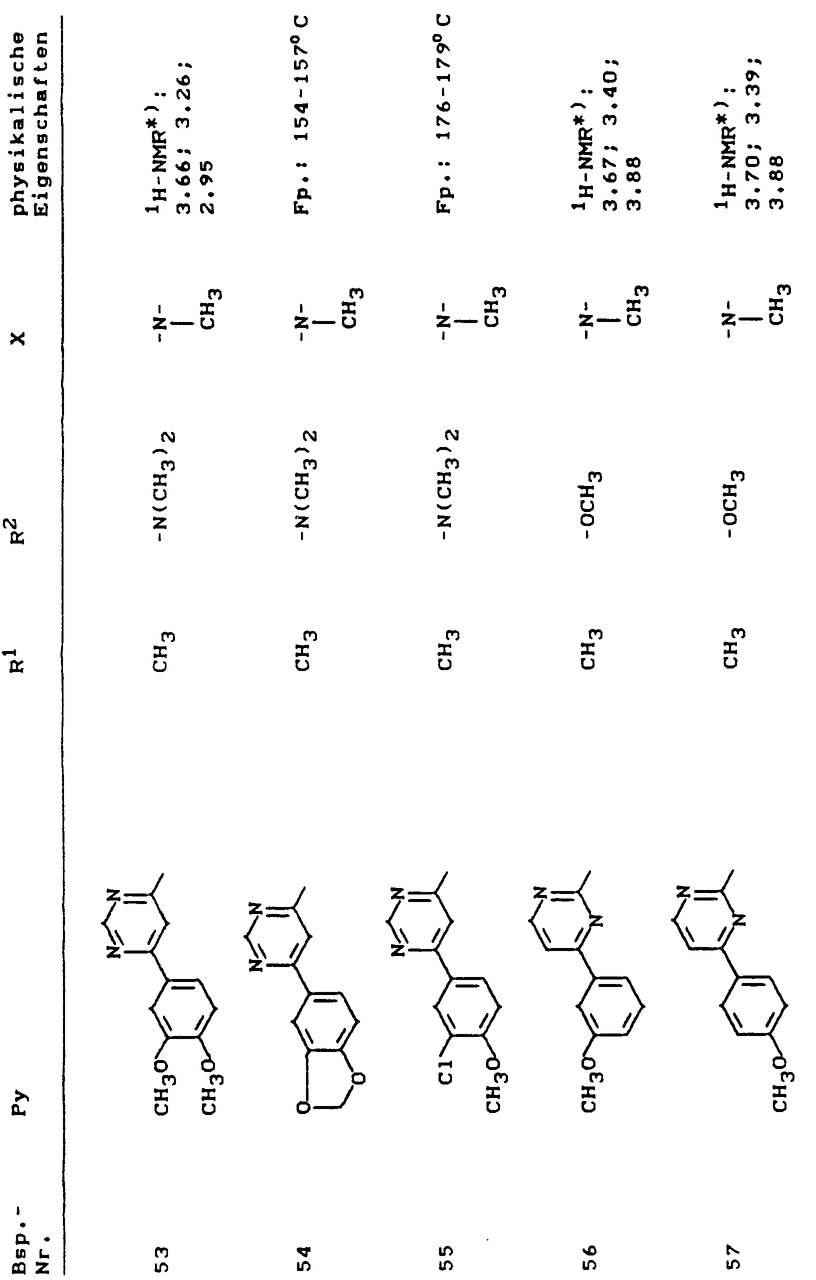

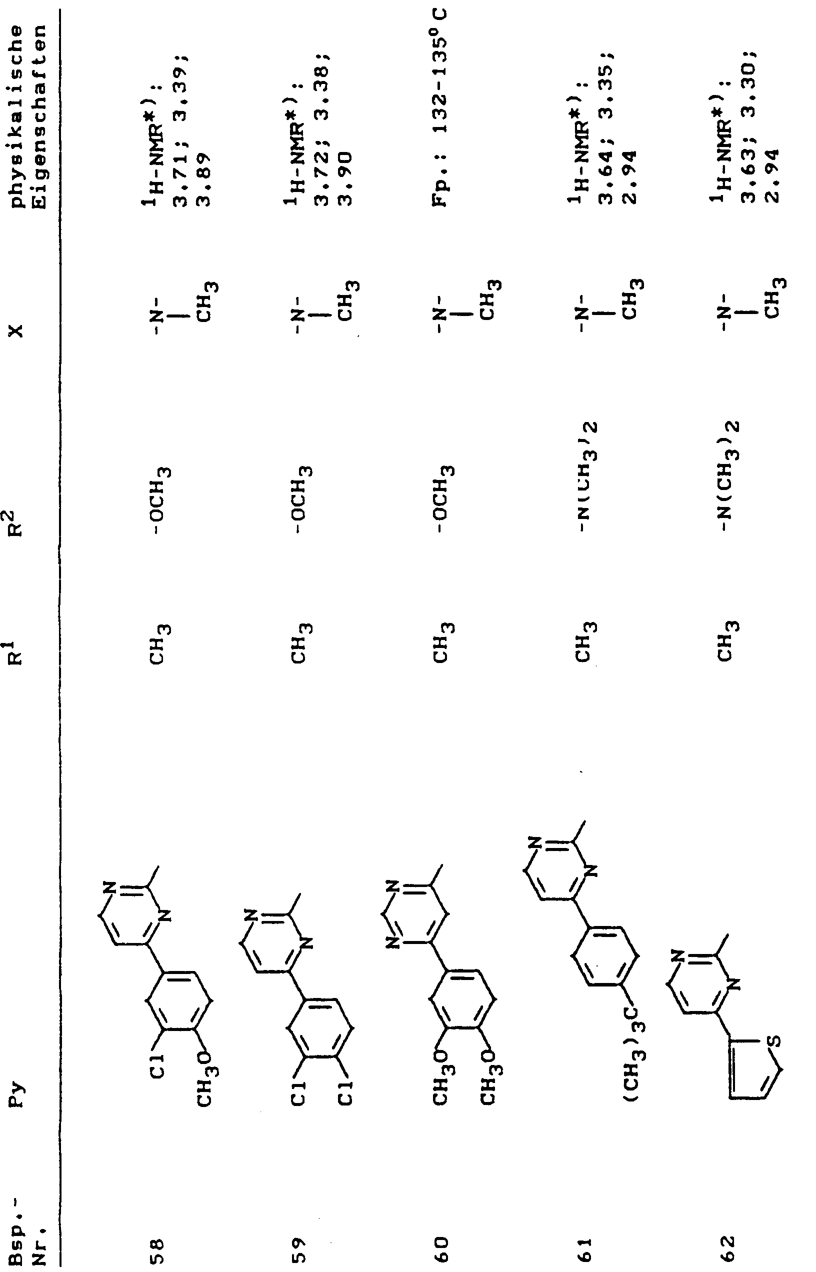

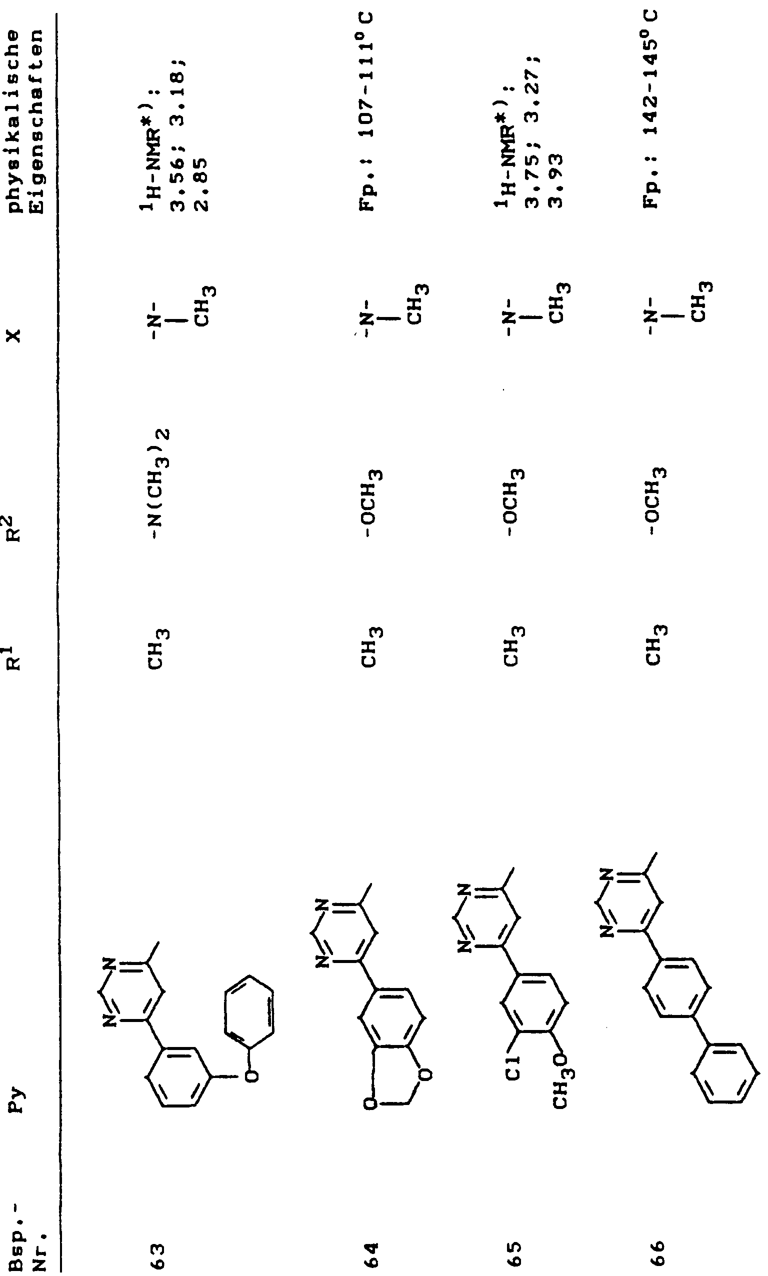

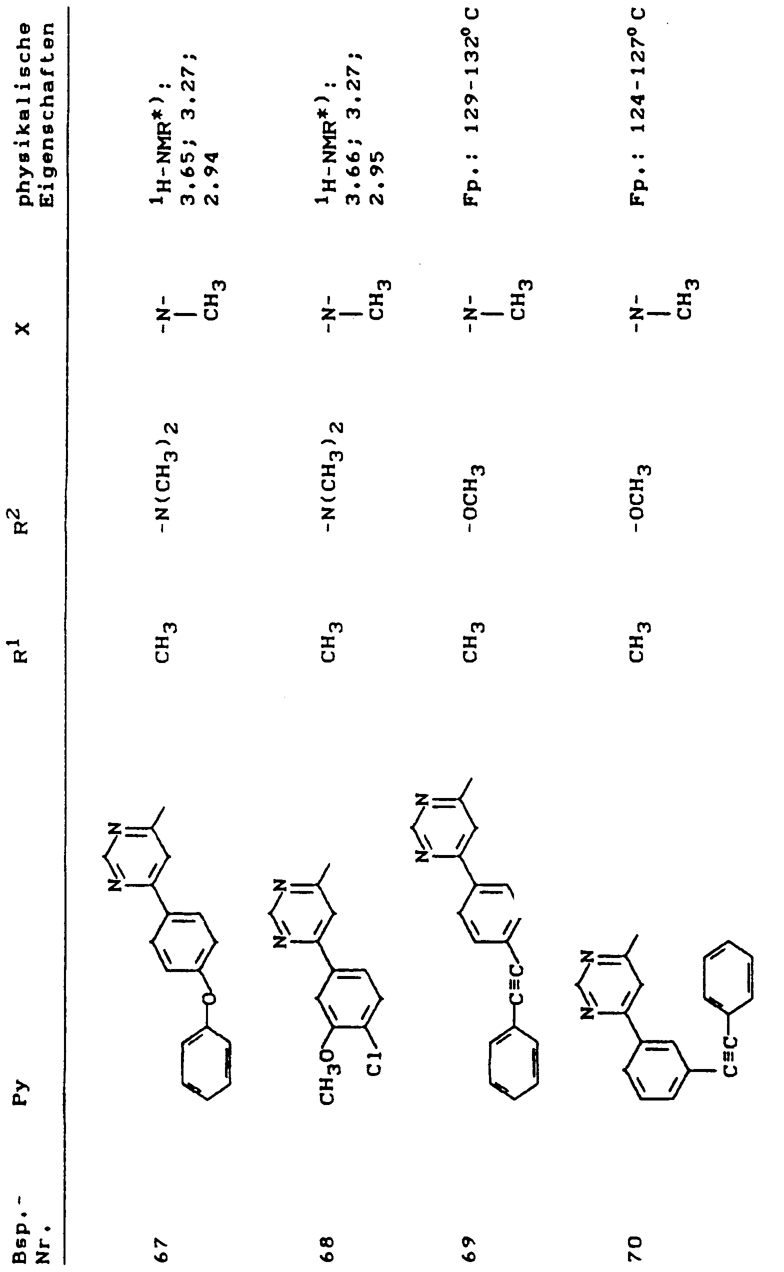

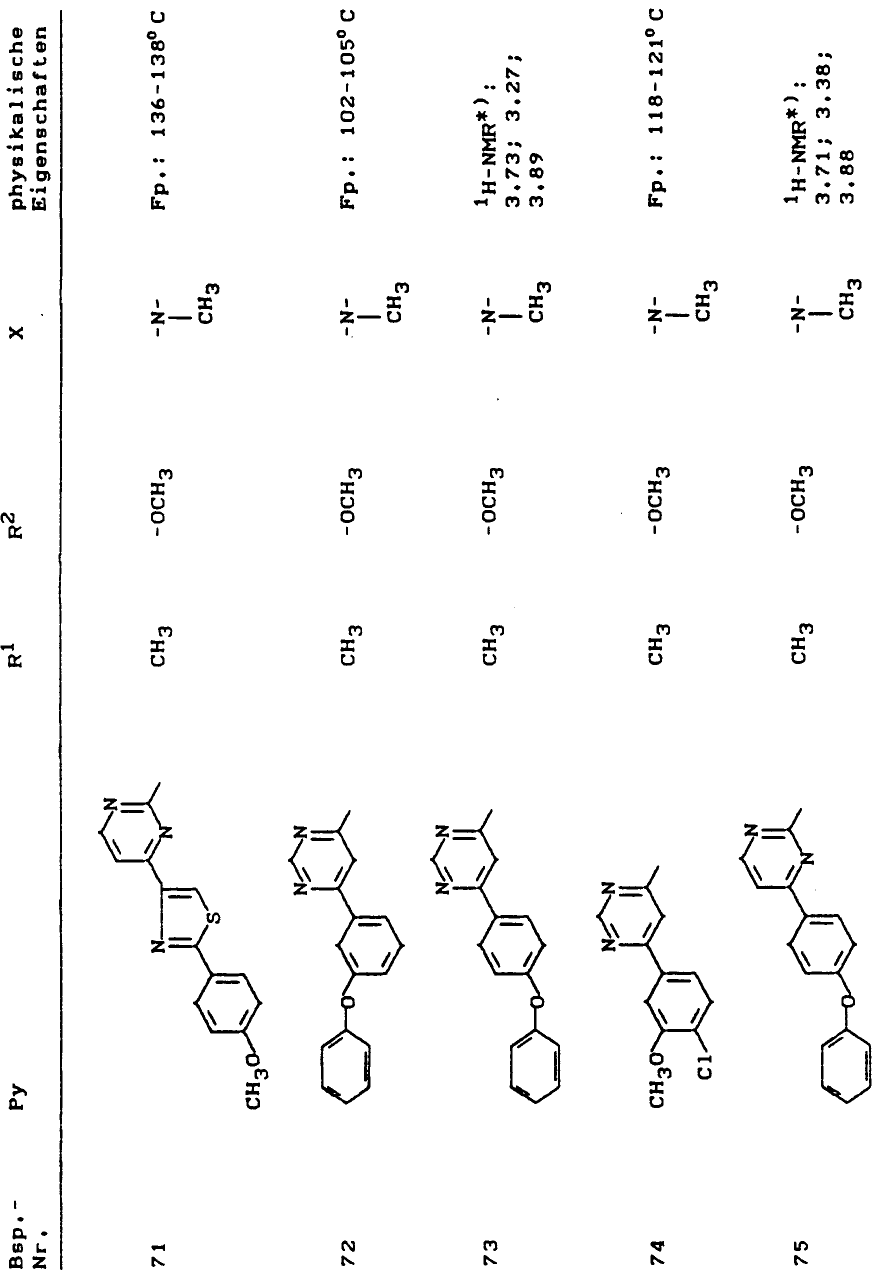

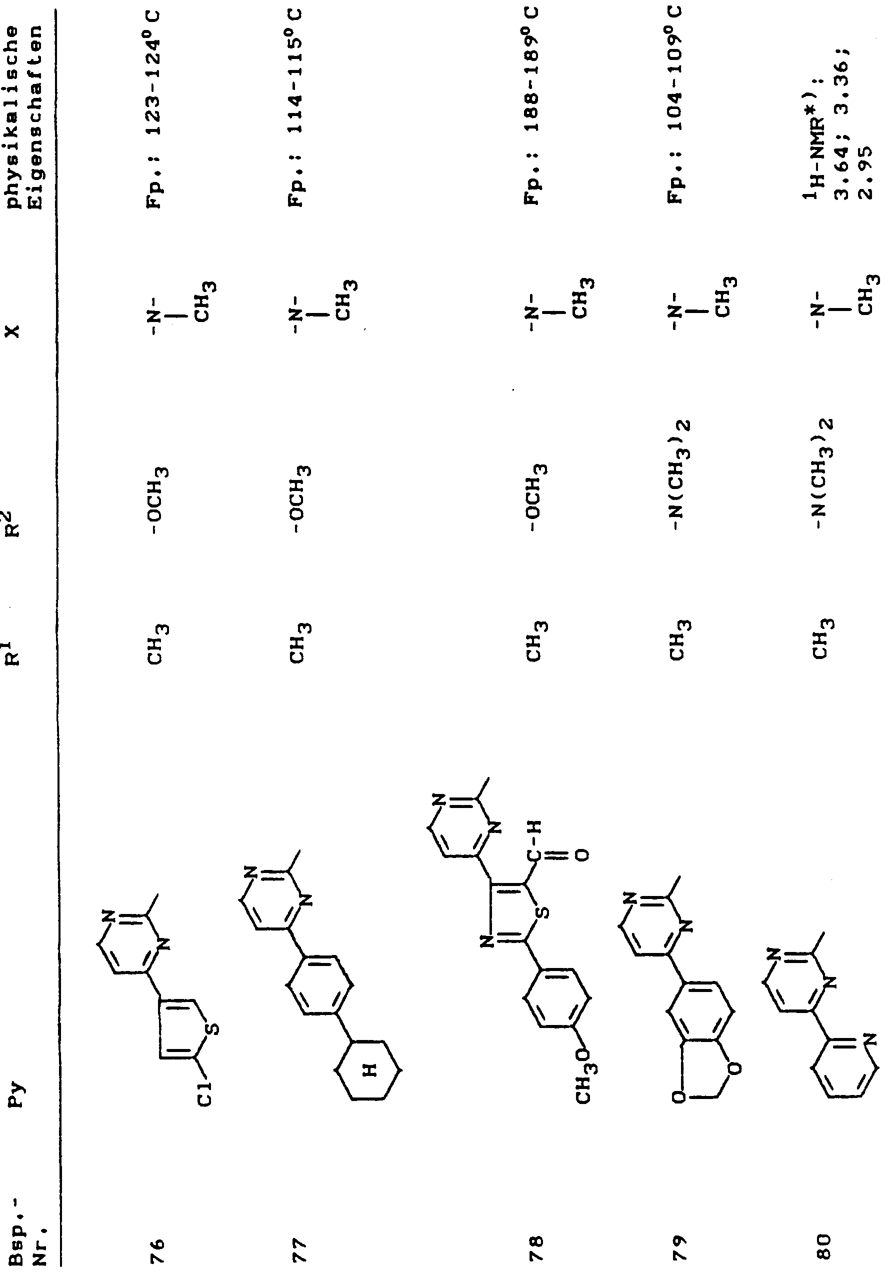

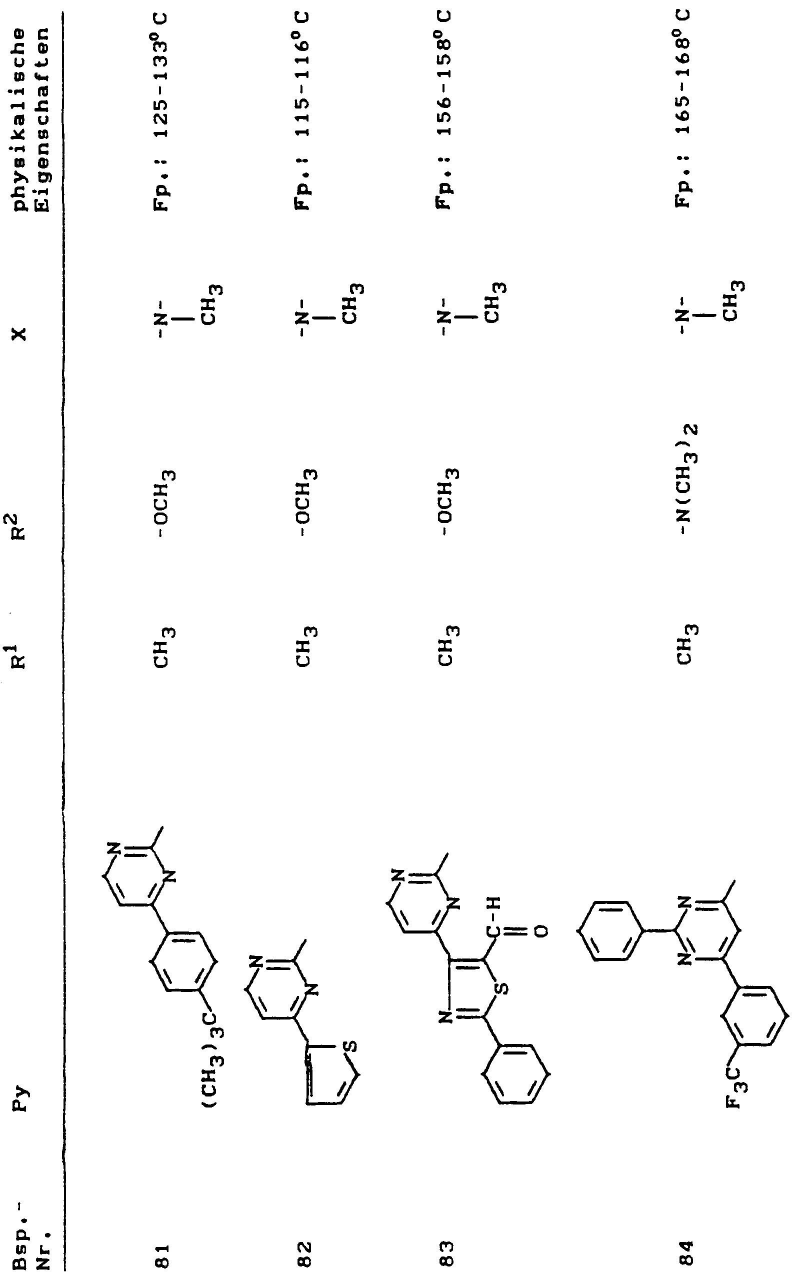

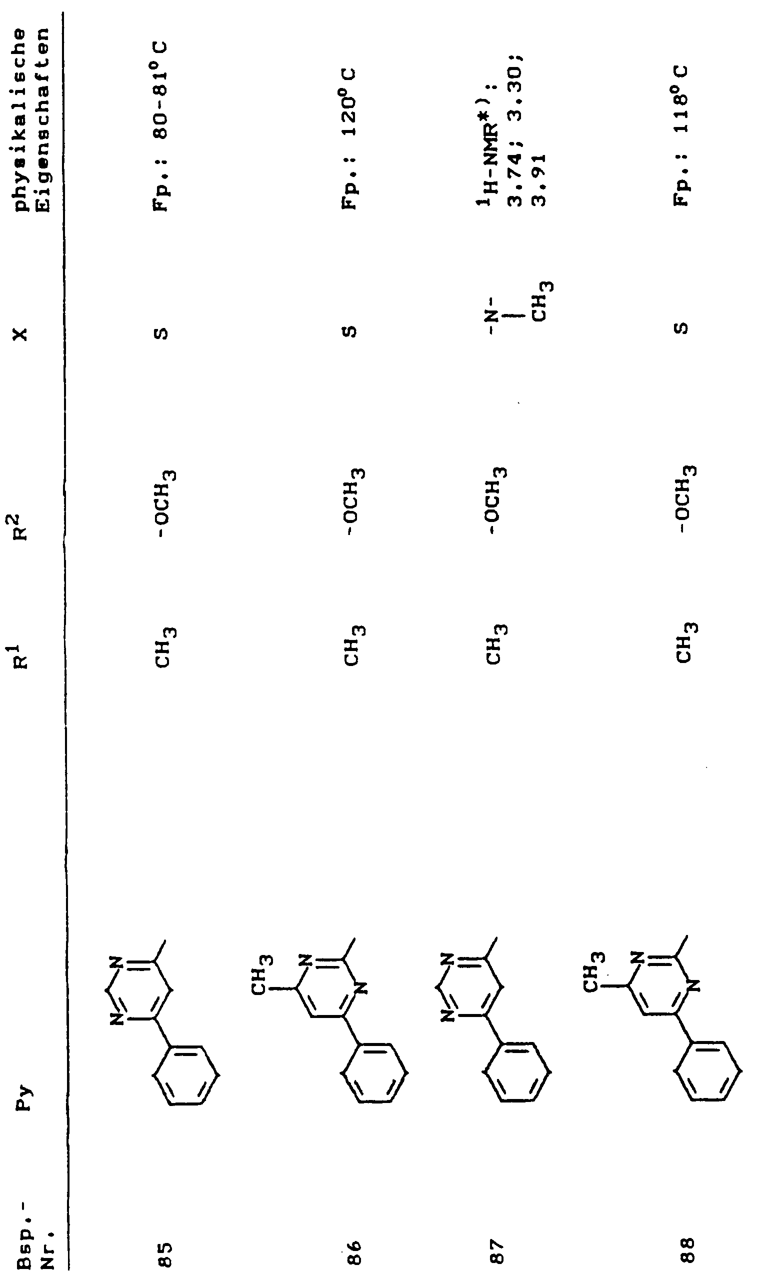

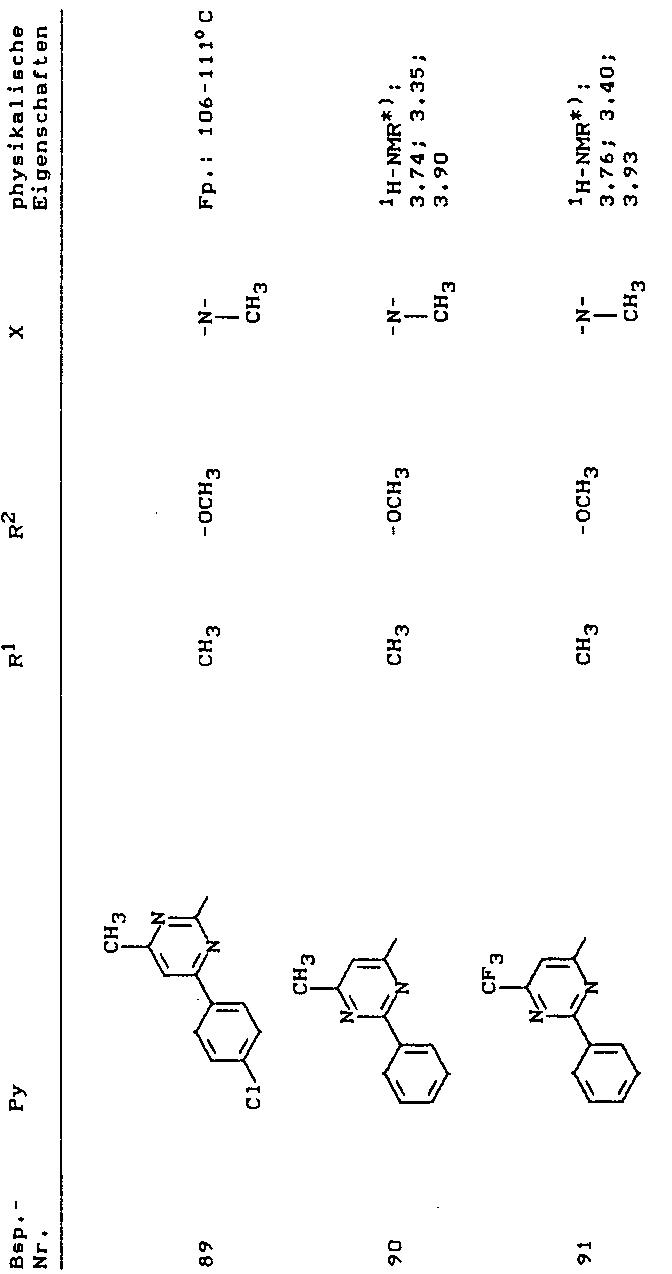

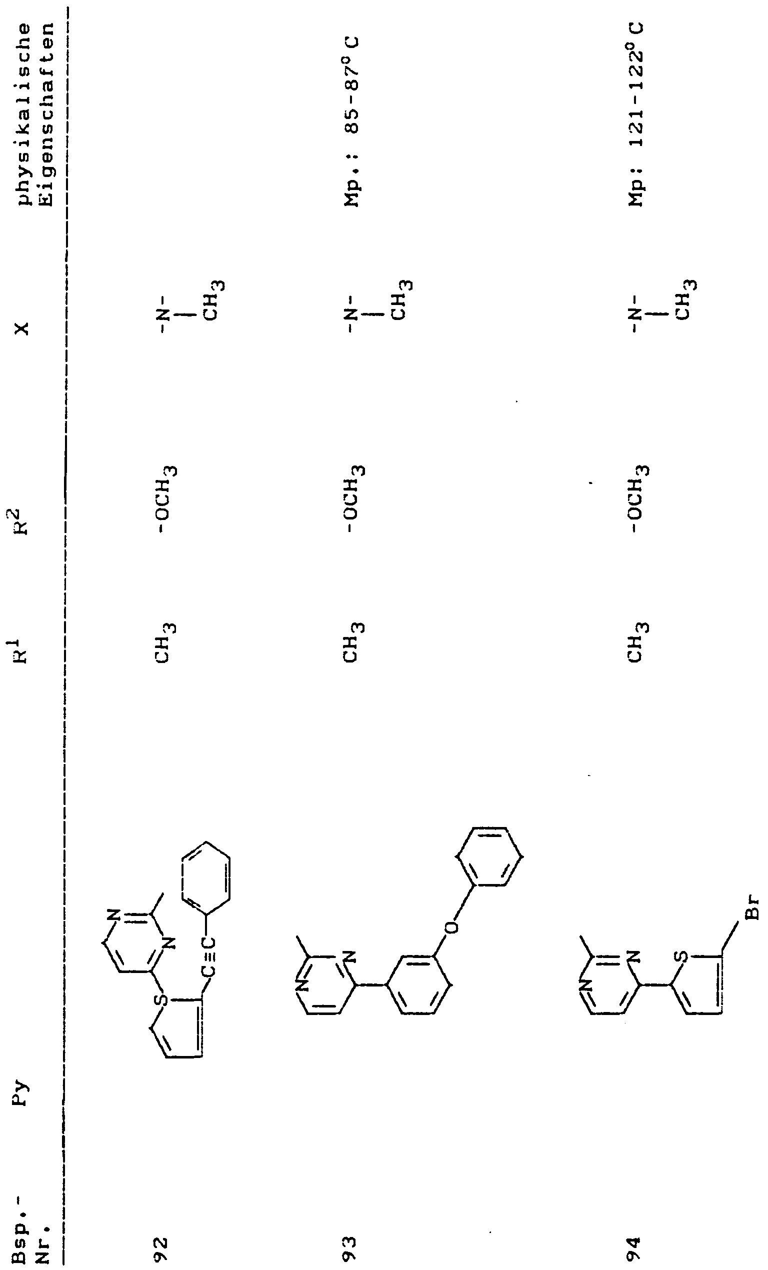

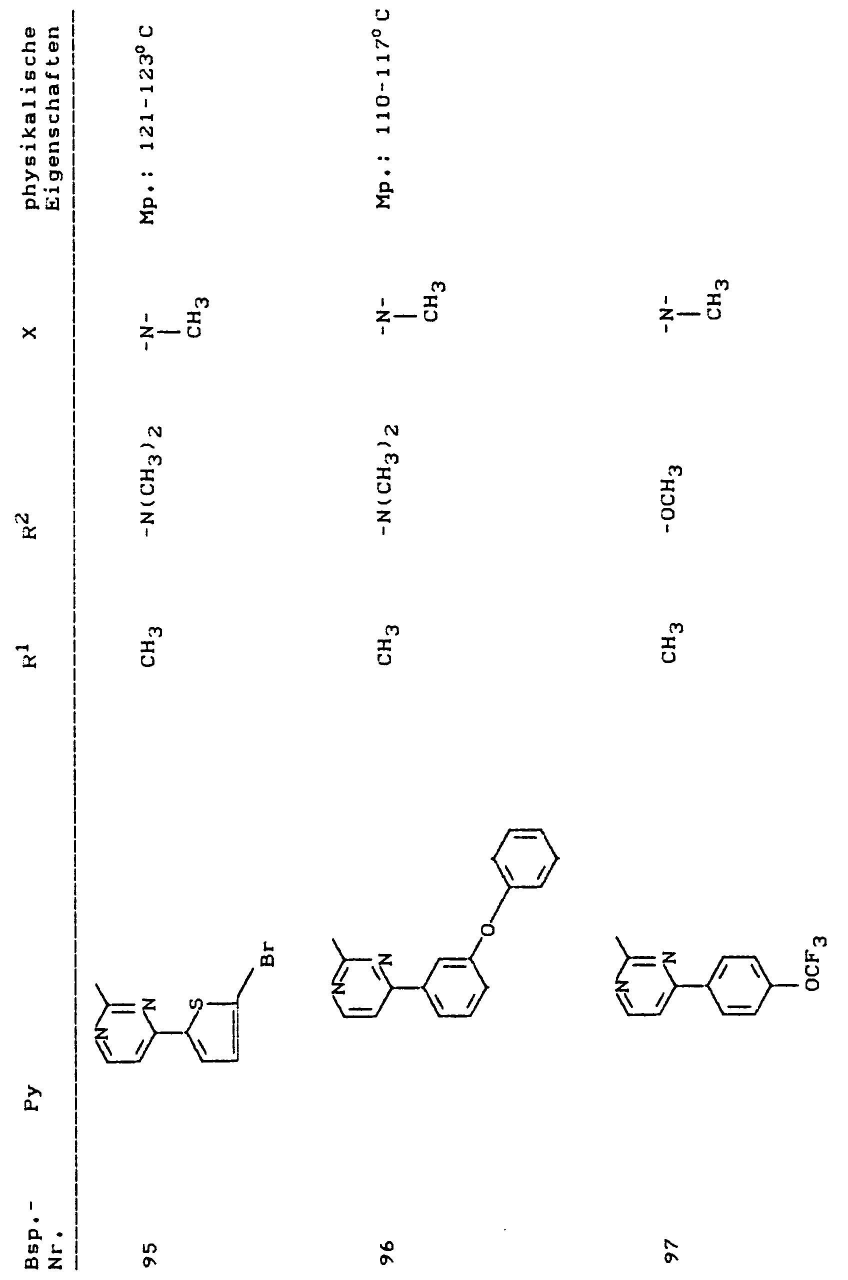

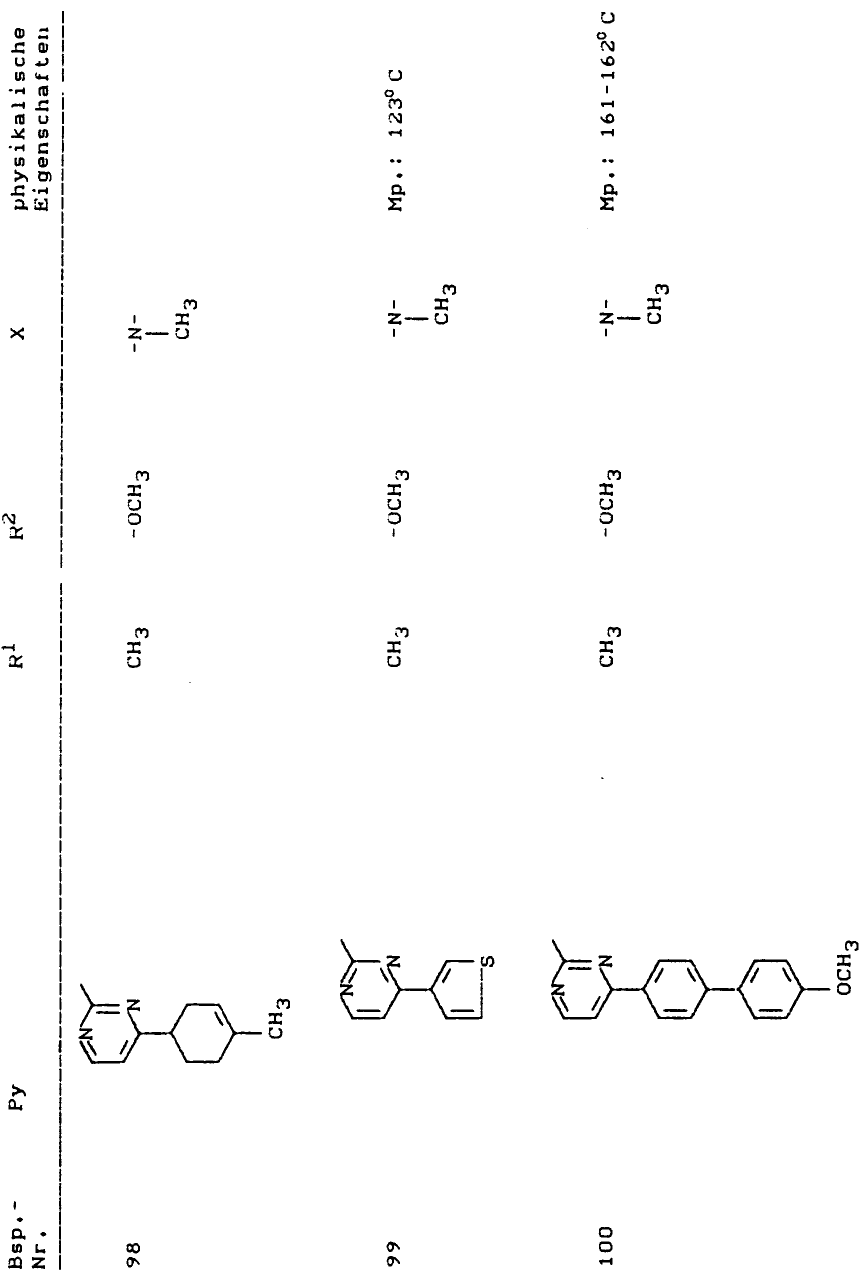

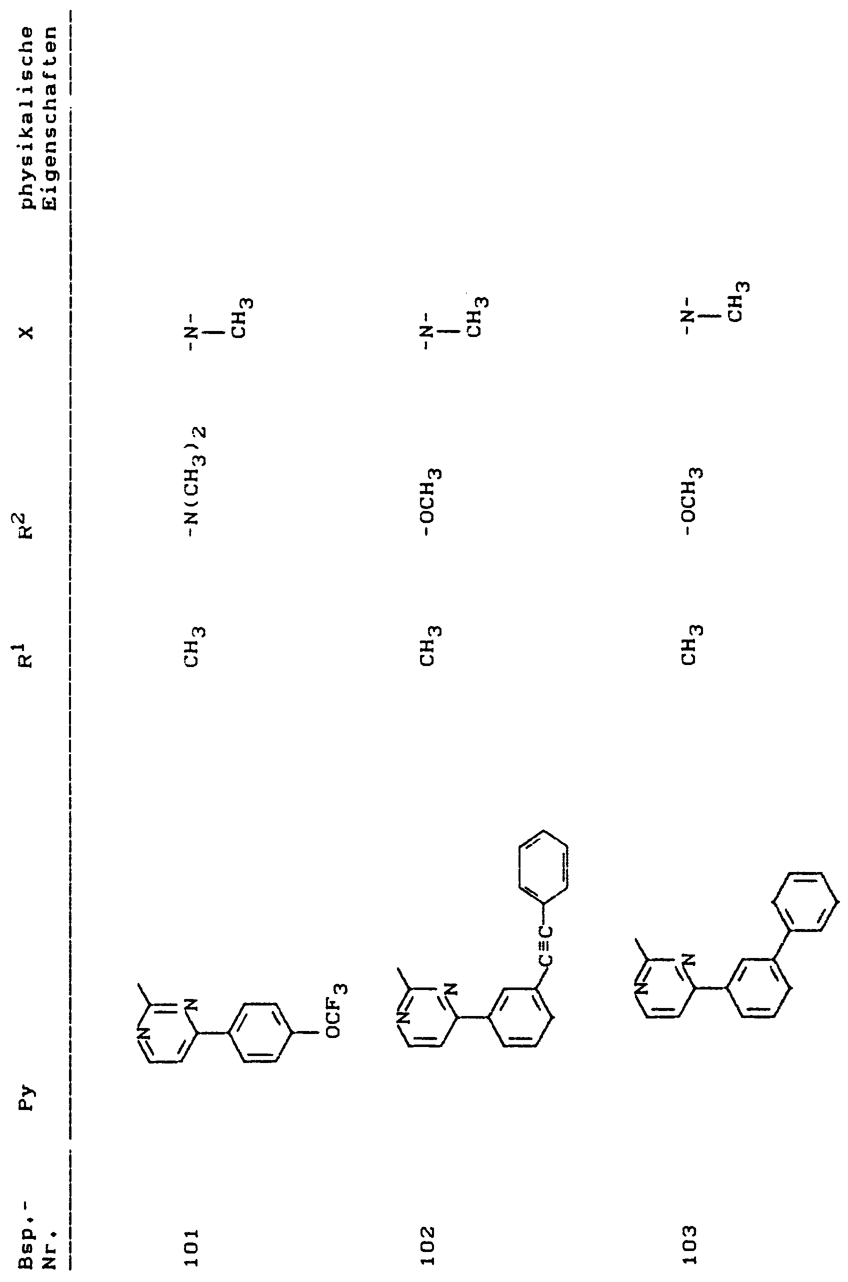

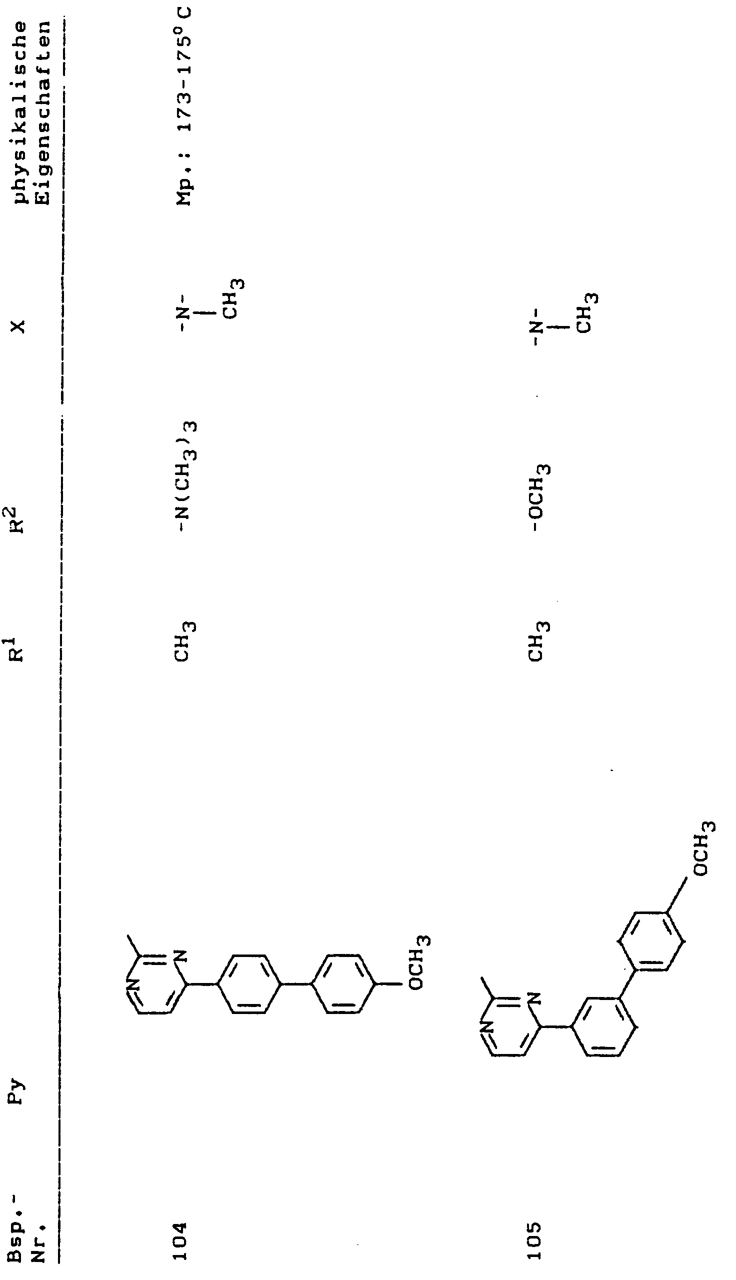

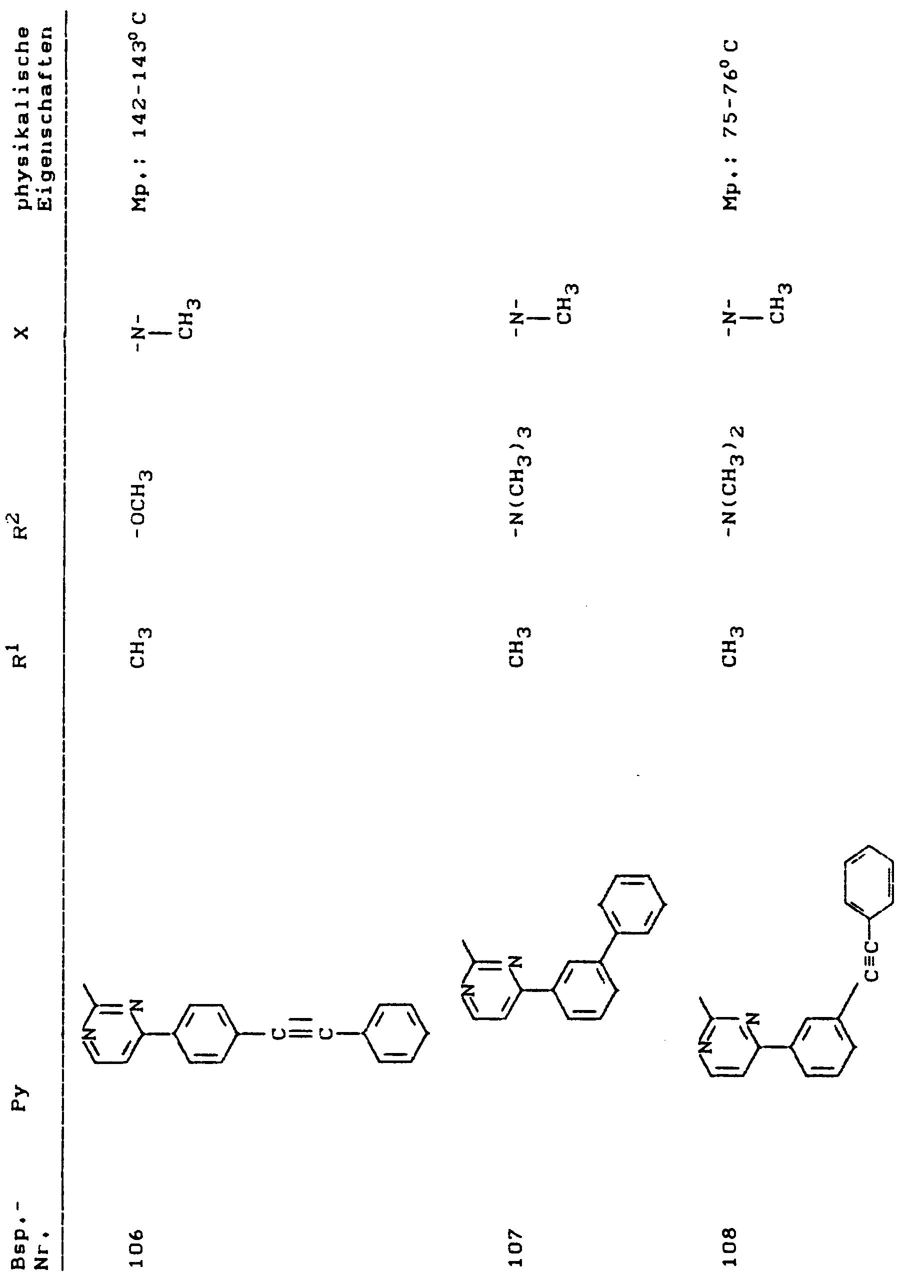

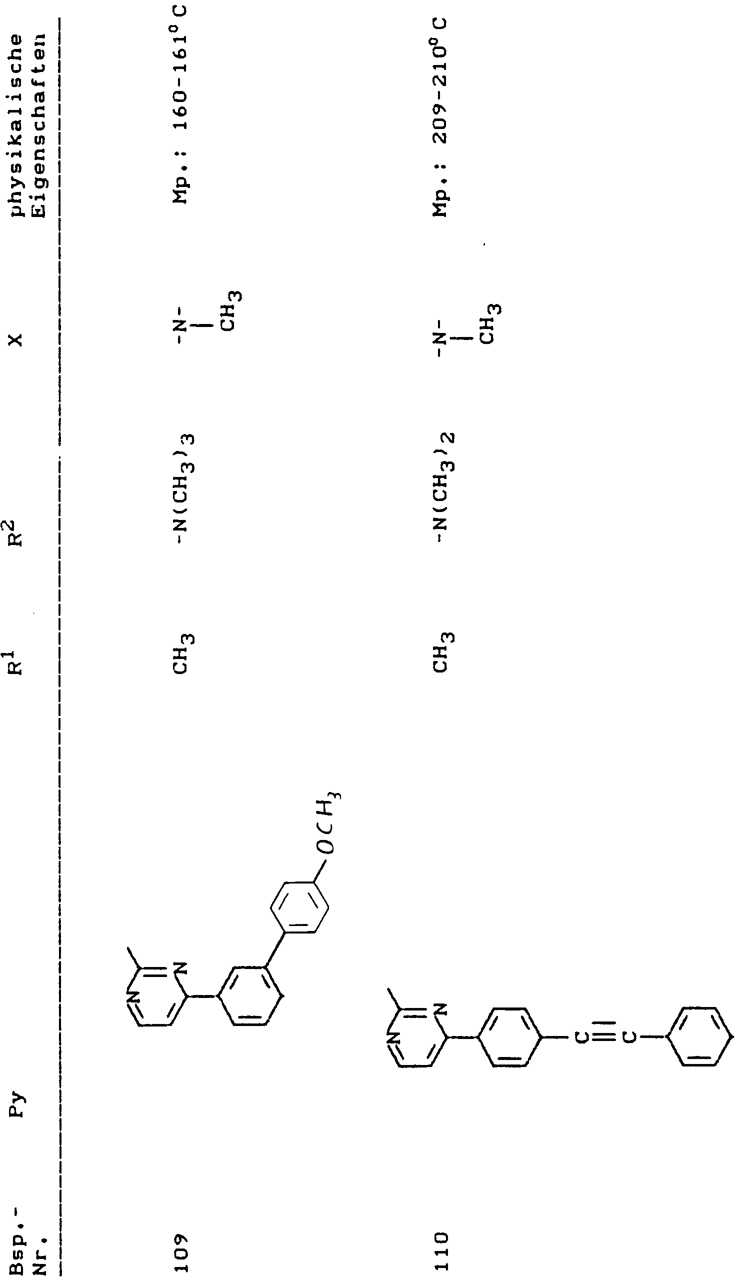

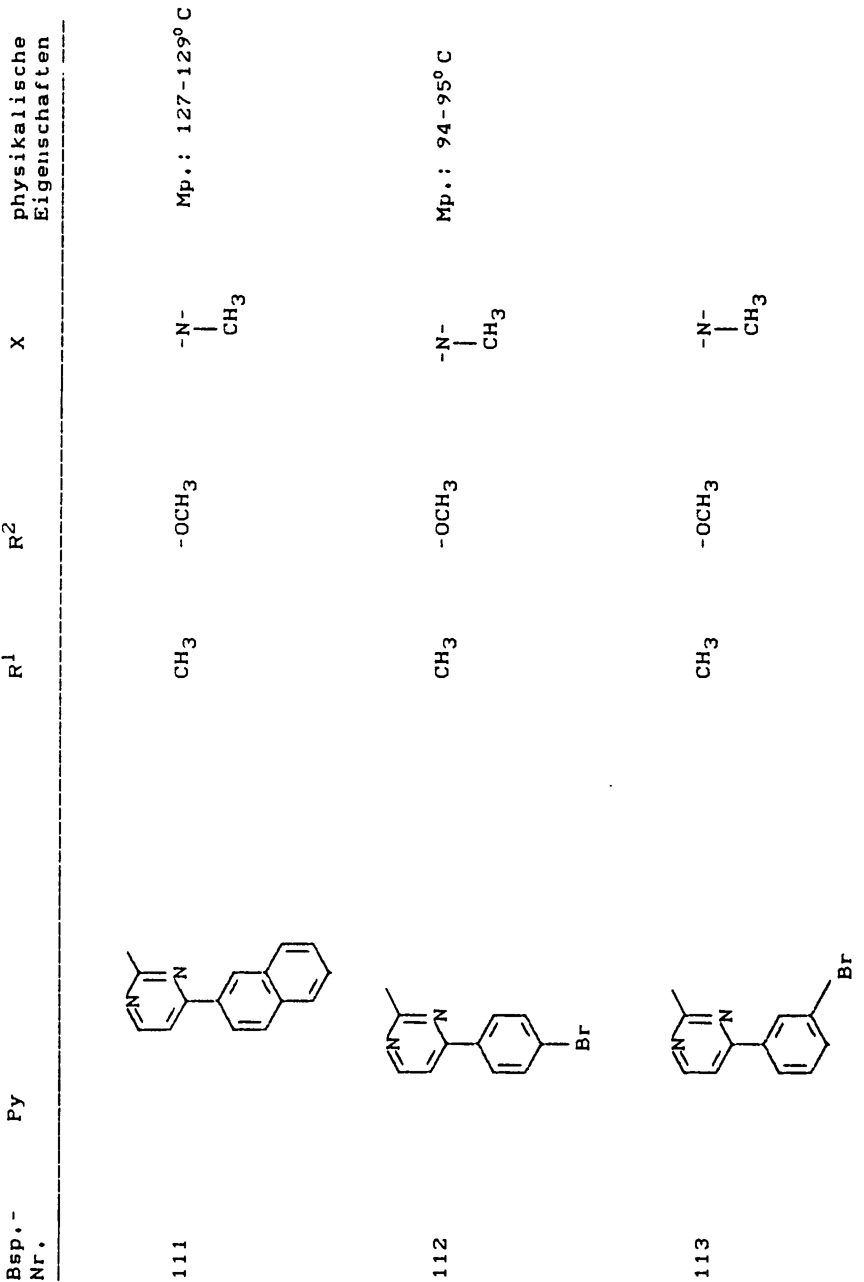

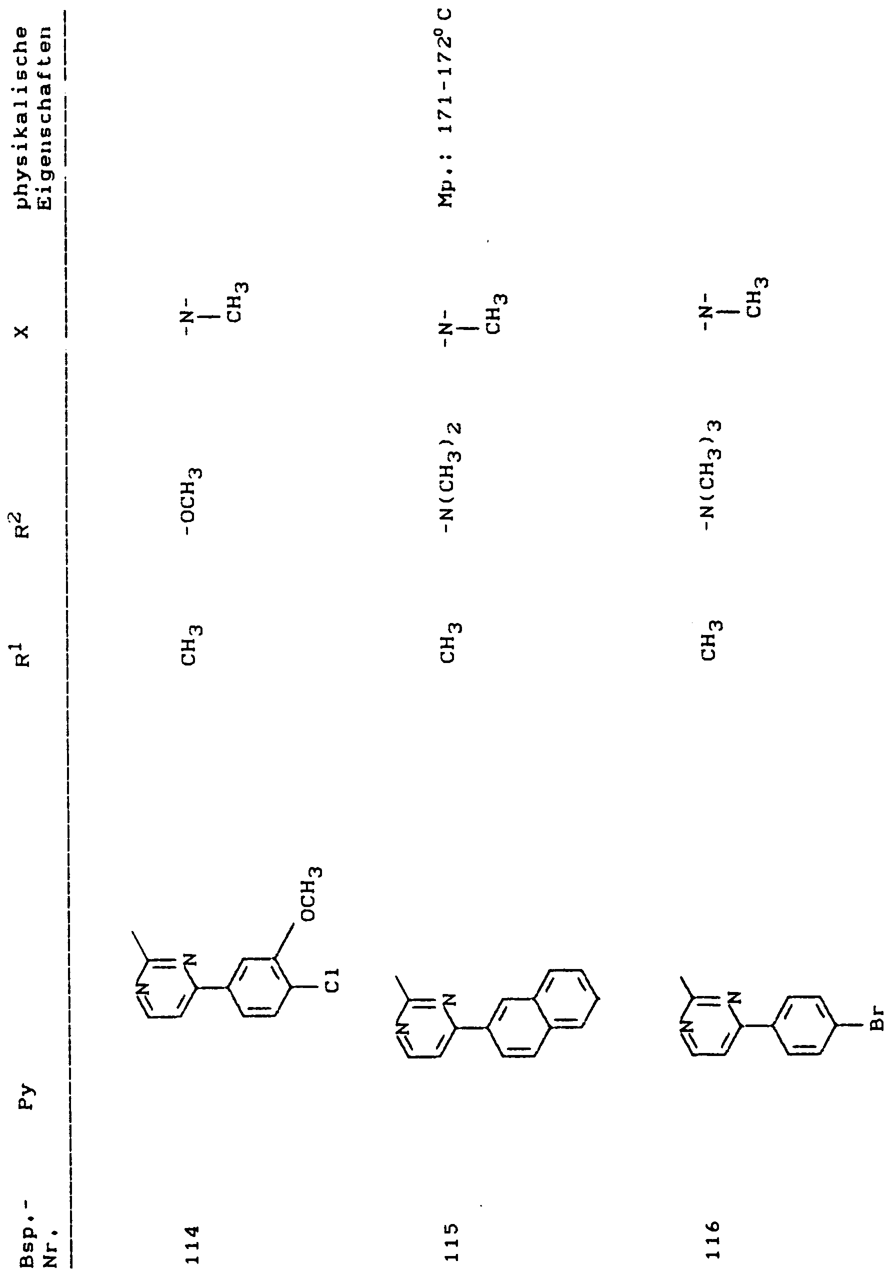

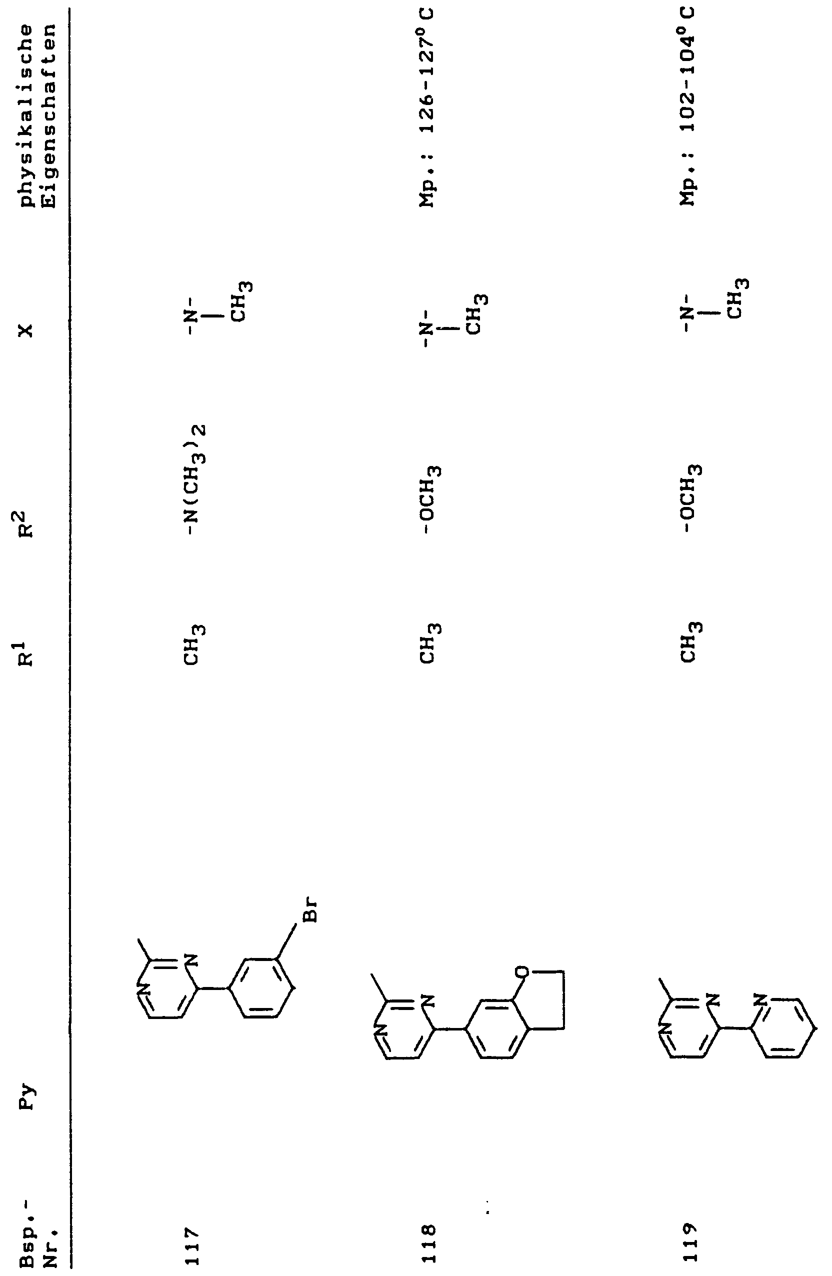

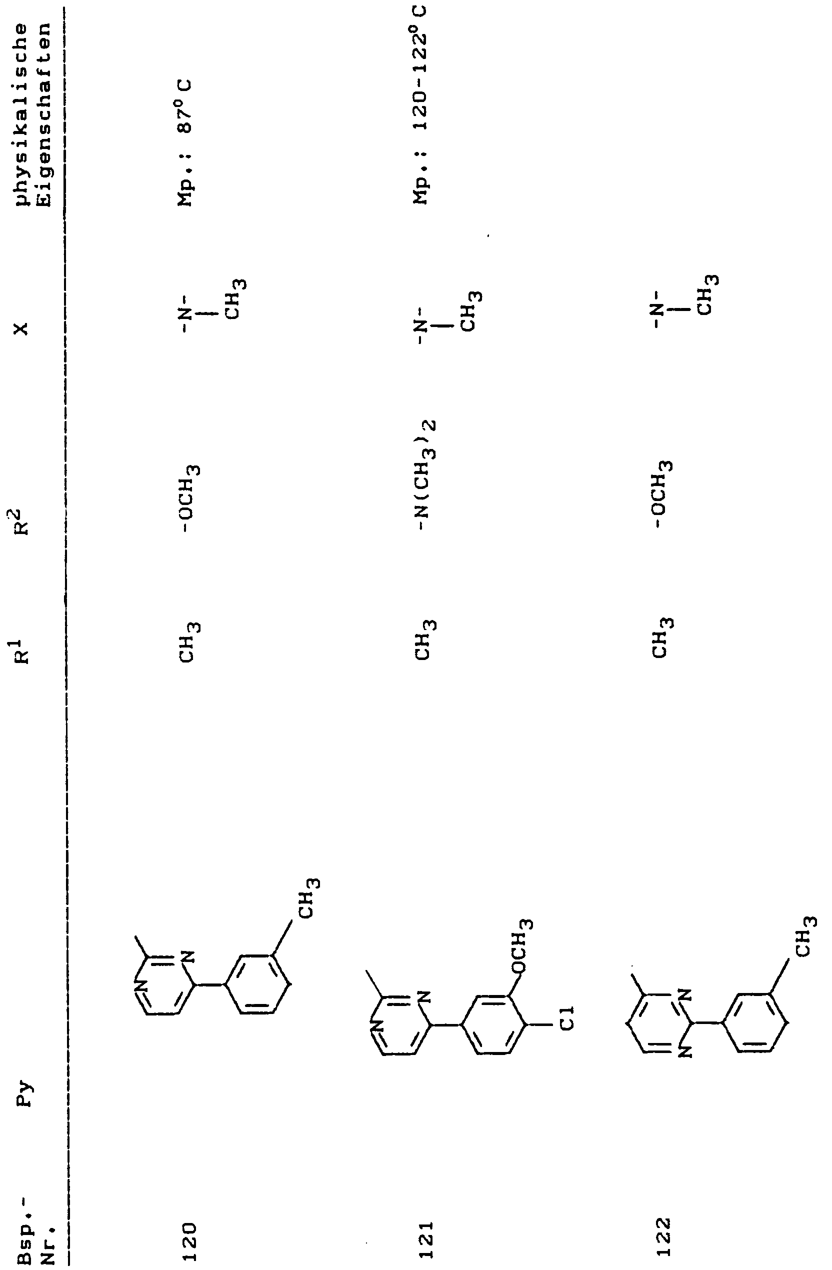

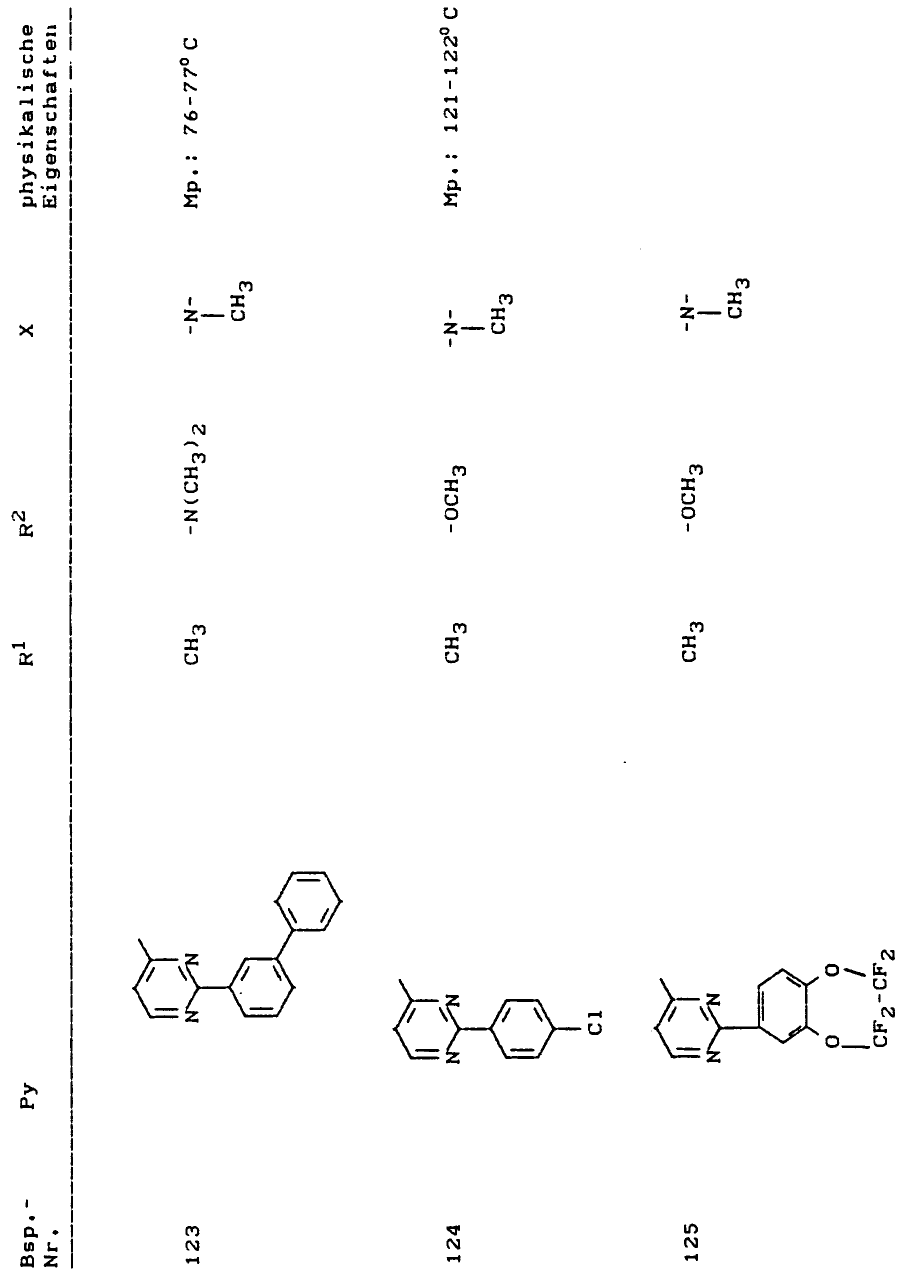

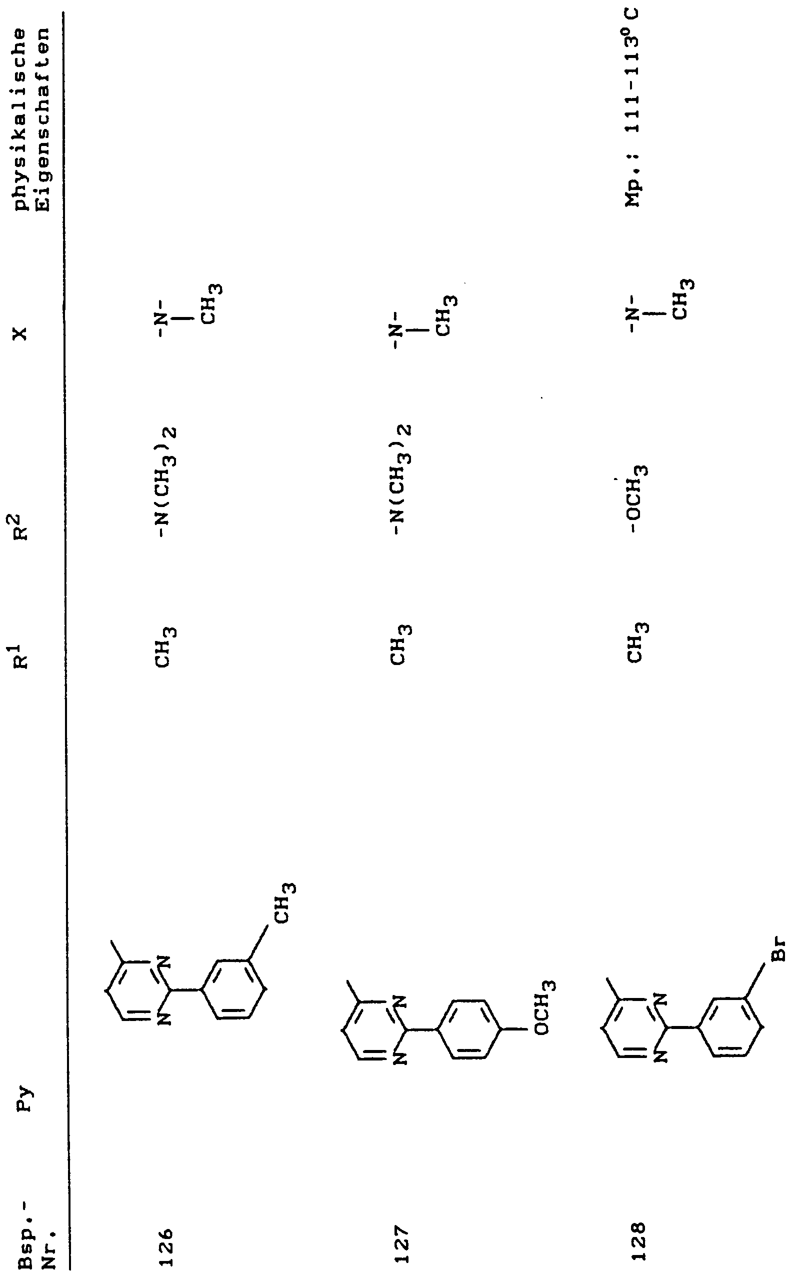

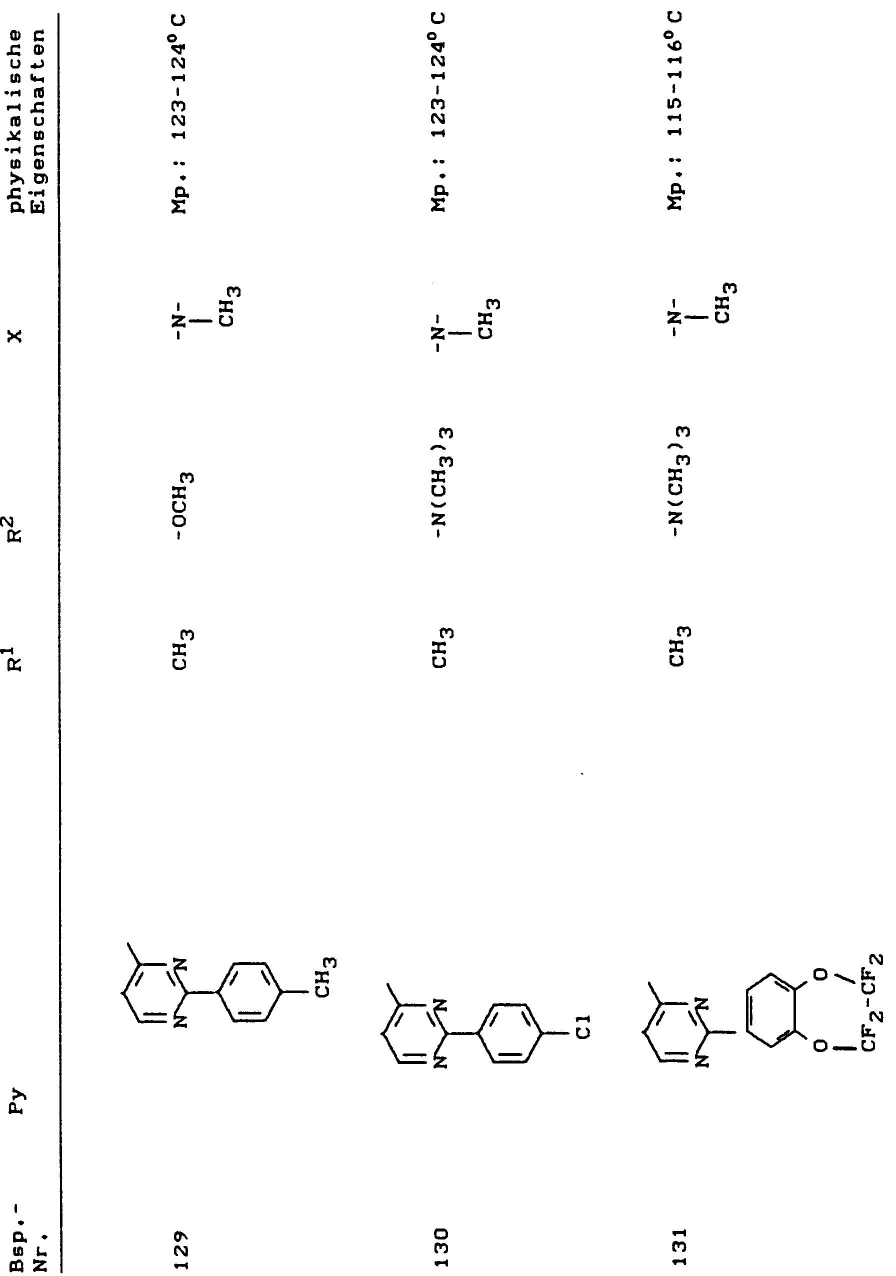

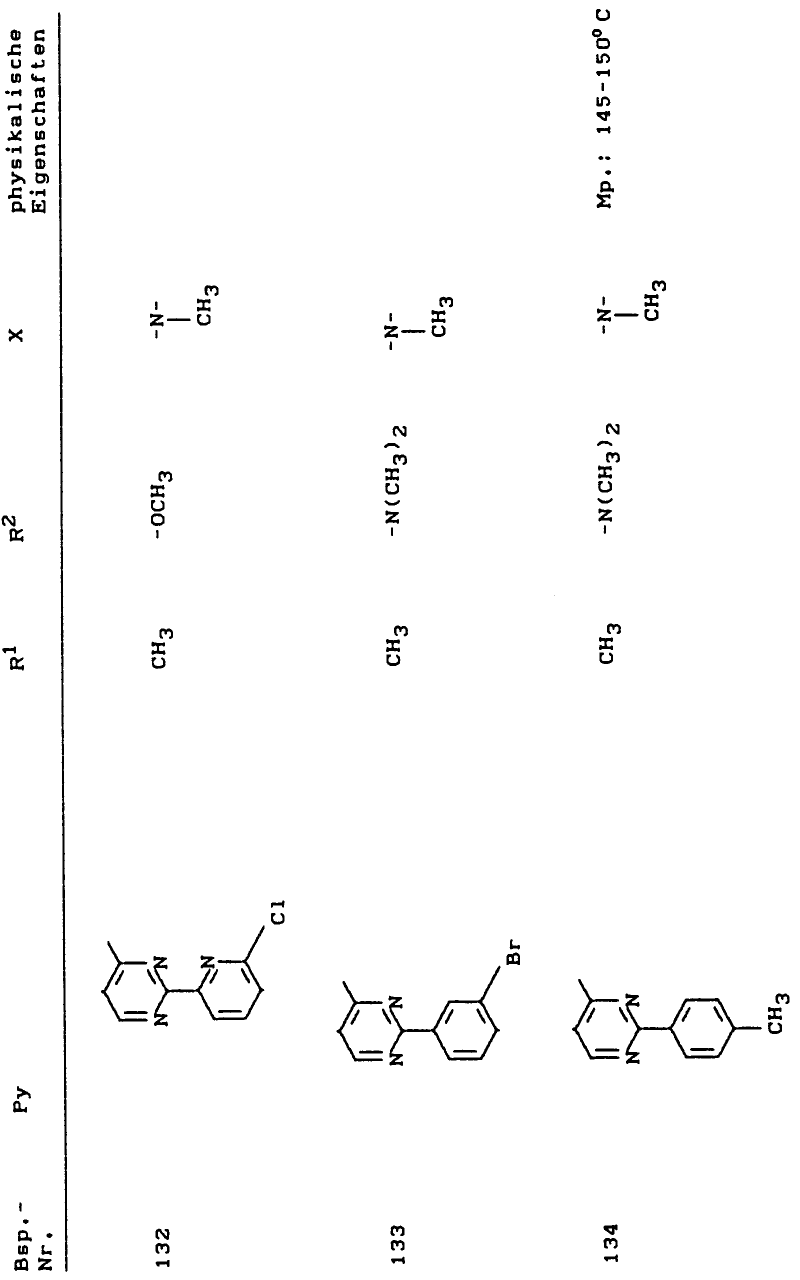

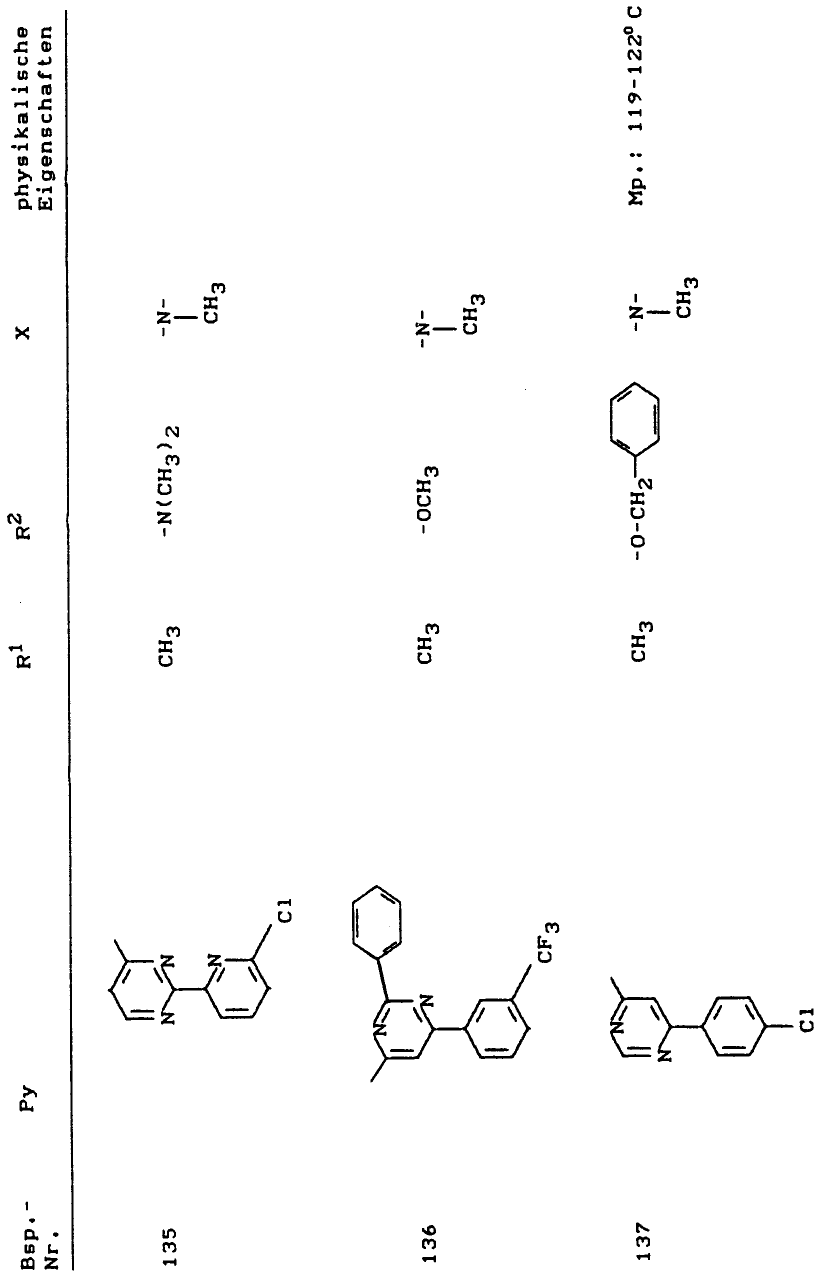

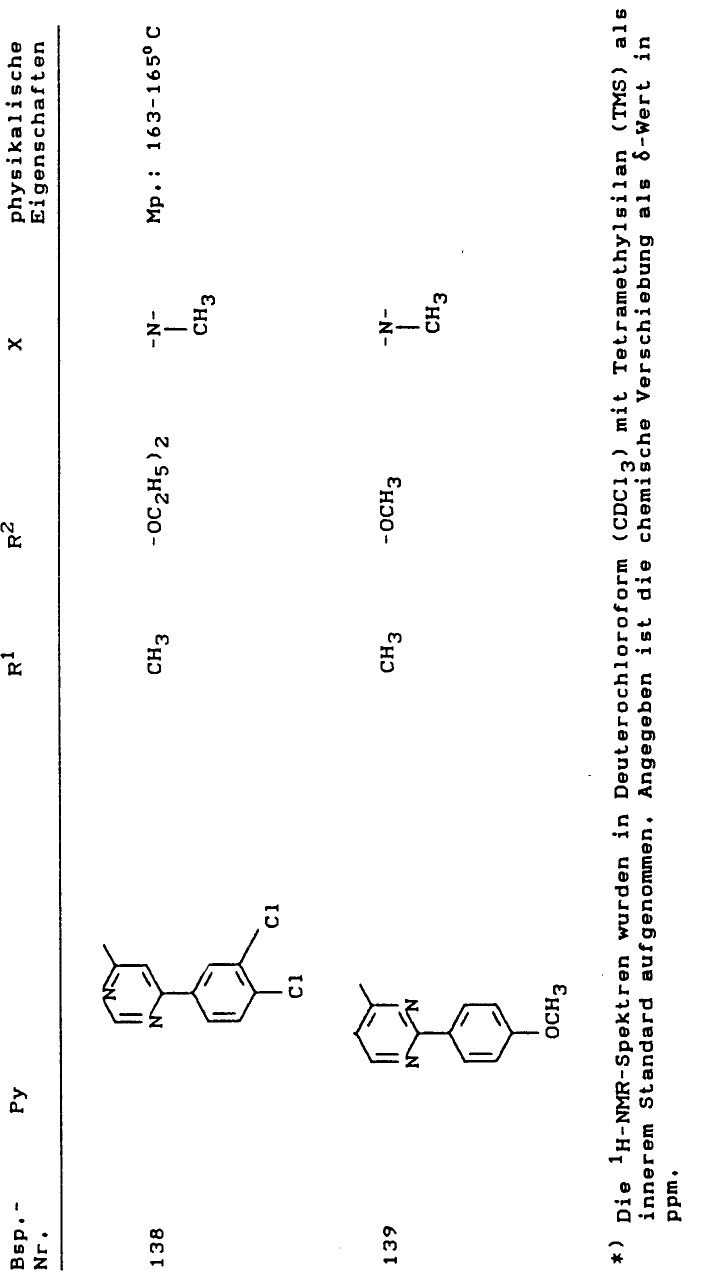

### Anwendungsbeispiele

In den folgenden Anwendungsbeispielen wurden die nachstehend aufgeführten Verbindungen als Vergleichssubstanzen eingesetzt: 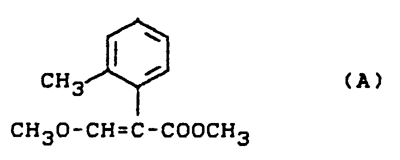
3-Methoxy-2-(2-methylphenyl)-acrylsäuremethylester (bekannt aus EP 178 826) 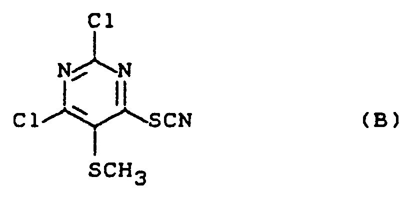
2,4-Dichlor-5-methylthiopyrimidinyl-6-thiocyanat (bekannt aus US-PS 4.652.569).

### Beispiel A

### Erysiphe-Test (Gerste) / protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 16, 45, 49, 50, 51.

Die Vergleichsverbindung (B) ist nicht wirksam.

### Beispiel B

### Leptosphaeria nodorum-Test (Weizen)/protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine bis zu 70 Wirkungsgrade (gemessen in %) bessere Wirksamkeit gegenüber der bekannten Verbindung (A) zeigen bei diesem Test z.B. die Verbindungen gemäß folgender Herstellungsbeispiele: 16 und 17.

### Beispiel C

### Cochliobolus sativus-Test (Gerste)/protektiv

- Lösungsmittel:: 100 Gewichtsteile Dimethylformamid
- Emulgator:: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 16, 17 und 49.

### Beispiel D

### Phytophthora-Test (Tomate)/protektiv

- Lösungsmittel:: 4,7 Gewichtsteile Aceton
- Emulgator:: 0,3 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 45, 46, 47, 49, 50, 51 und 73.

### Beispiel E

### Plasmopara-Test (Reben) / protektiv

- Lösungsmittel:: 4,7 Gewichtsteile Aceton
- Emulgator:: 0,3 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 45, 50, 51, 65, 66, 73, 87, 89 und 90.

## Patentansprüche

1. Pyrimidyl-substituierte Acrylsäureester der allgemeinen Formel (I) 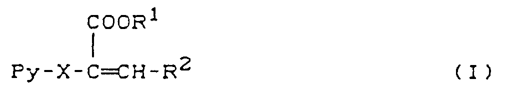 in welcher
R¹ für geradkettiges oder verzweigtes Alkyl mit 1 bis 6 Kohlenstoffatomen steht,
R² für Dialkylamino mit jeweils 1 bis 6 Kohlenstoffatomen in den einzelnen Alkylteilen steht oder für einen Rest -O-R³ steht,
X für Sauerstoff, Schwefel oder für einen Rest 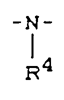 steht und
Py für jeweils einfach oder mehrfach, gleich oder verschieden substituiertes 2-Pyrimidyl, 4-Pyrimidyl oder 5-Pyrimidyl steht, wobei als Substituenten jeweils infrage kommen: Halogen, Cyano, Nitro, jeweils geradkettiges oder verzweigtes Alkyl, Alkoxy oder Alkylthio mit jeweils 1 bis 9 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, jeweils gegebenenfalls einfach oder mehrfach durch C₁-C₄-Alkyl substituiertes Cycloalkyl oder Cycloalkenyl mit jeweils 3 bis 7 Kohlenstoffatomen, zweifach verknüpftes Alkandiyl mit 3 bis 5 Kohlenstoffatomen oder jeweils im Arylteil oder im Heteroarylteil gegebenenfalls einfach oder mehrfach gleich oder verschieden durch
- Halogen, Alkyl, Cycloalkyl, Alkoxy, Alkylthio, Halogenalkyl, Halogenalkoxy, Halogenalkylthio, Formyl, Dioxyalkylen, halogensubstituiertes Dioxyalkylen oder jeweils gegebenenfalls substituiertes Phenyl, Phenoxy, Benzyl, Phenylethyl, Phenylethenyl oder Phenylethinyl
substituiertes Aryl, Aryloxy, Arylthio, Arylamino, N-Alkyl-arylamino, Arylcarbonyl, Aralkyl, Arylalkenyl, Arylalkinyl, Arylalkyloxy, Arylalkylthio, Heteroaryl, Heteroaryloxy, Heteroarylthio, Heteroarylamino, N-Alkyl-heteroarylamino, Heteroarylcarbonyl, Heteroarylalkyl, Heteroarylalkenyl, Heteroarylalkinyl, Heteroarylalkyloxy oder Heteroarylalkylthio mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenyl-bzw. Alkinylteil;
wobei
R³ für geradkettiges oder verzweigtes Alkyl mit 1 bis 6 Kohlenstoffatomen oder für Aralkyl mit 1 bis 4 Kohlenstoffatomen im geradkettigen oder verzweigten Alkylteil und 6 bis 10 Kohlenstoffatomen im Arylteil steht, und
R⁴ für Wasserstoff, für geradkettiges oder verzweigtes Alkyl mit 1 bis 6 Kohlenstoffatomen oder für jeweils gegebenenfalls im Arylteil einfach oder mehrfach, gleich oder verschieden substituiertes Aralkyl oder Aryl mit 1 bis 6 Kohlenstoffatomen im geradkettigen oder verzweigten Alkylteil und 6 bis 10 Kohlenstoffatomen im jeweiligen Arylteil steht, wobei als Substituenten im Arylteil jeweils die bei Py genannten infrage kommen.

2. Pyrimidyl-substituierte Acrylsäureester der Formel (I) gemäß Anspruch 1, bei welchen
R¹ für Methyl, Ethyl, n- oder i-Propyl, n-, i-, s- oder t-Butyl steht,
R² für Dialkylamino mit jeweils 1 bis 4 Kohlenstoffatomen in den einzelnen Alkylteilen steht oder für einen Rest -O-R³ steht,
X für Sauerstoff, Schwefel oder für einen Rest 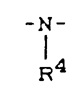 steht und
Py für jeweils ein- bis dreifach, gleich oder verschieden substituiertes 2-Pyrimidyl oder 4-Pyrimidyl steht, wobei mindestens ein Substituent jeweils für gegebenenfalls ein- bis dreifach, gleich oder verschieden
- durch Fluor, Chlor, Brom, Methyl, Ethyl, n-oder i-Propyl, n-, i-, s- oder t-Butyl, Methoxy, Ethoxy, n- oder i-Propoxy, Methylthio, Ethylthio, Trifluormethyl, Trifluormethoxy, Formyl, Trifluormethylthio, Dioxymethylen, Dioxyethylen, Tetrafluordioxyethylen, Difluordioxymethylen, Cyclopentyl, Cyclohexyl oder durch jeweils gegebenenfalls ein- bis dreifach, gleich oder verschieden [durch Fluor, Chlor, Methyl, Methoxy oder Trifluormethyl] substituiertes Phenyl, Phenoxy, Benzyl, Phenylethyl, Phenylethenyl oder Phenylethinyl -
substituiertes Phenyl, Naphthyl, Phenoxy, Phenylthio, N-Methyl-phenylamino, Phenylcarbonyl, Benzyl, Phenylethyl, Phenylpropyl, Phenylethenyl, Phenylethinyl, Benzyloxy, Cyclohexenyl oder Heteroaryl steht, wobei als Heteroarylreste im einzelnen die folgenden infrage kommen: 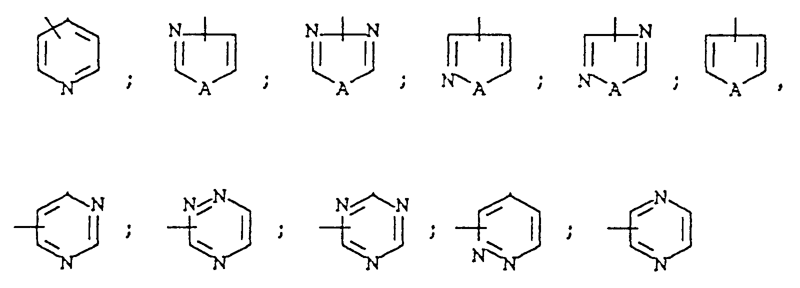 welche gegebenenfalls auch benzanelliert sein können und bei welchen
A jeweils für Sauerstoff, Schwefel oder eine NH-Gruppe steht;
darüberhinaus kommen als weitere Pyrimidyl-substituenten vorzugsweise die folgenden infrage:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trichlormethoxy, Trifluormethylthio, Methoxycarbonyl, Ethoxycarbonyl, Methoximinomethyl, Ethoxzminomethyl, Methoximinoethyl, Ethoximinoethyl, Dimethylamino, Diethylamino, Dimethylcarbamoyl, Diethylcarbamoyl, Allyl, Butenyl, Ethinyl, Vinyl, Propargyl, Cyclopentyl, Cyclohexyl, 1,3-Propandiyl oder 1,4-Butandiyl;
wobei
R³ für Methyl, Ethyl, n- oder i-Propyl, n-, i-, s- oder t-Butyl oder für Benzyl steht und
R⁴ für Wasserstoff, Methyl, Ethyl, n- oder i-Propyl, n-, i-, s- oder t-Butyl oder für Benzyl oder Phenyl steht.

3. Pyrimidyl-substituierte Acrylsäureester der Formel (I) gemäß Anspruch 1, bei welchen
R¹ für Methyl oder Ethyl steht,
R² für Methoxy oder Ethoxy steht,
X für einen Rest 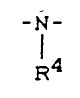 steht und
Py für jeweils ein- bis dreifach, gleich oder verschieden substituiertes 2-Pyrimidyl oder 4-Pyrimidyl steht, wobei mindestens ein Substituent jeweils für gegebenenfalls ein- bis dreifach, gleich oder verschieden durch Fluor, Chlor, Brom, Methyl, Ethyl, t-Butyl, Methoxy, Ethoxy, Trifluormethyl, Trifluormethoxy, Dioxymethylen, Difluordioxymethylen, Dioxyethylen, Tetrafluordioxyethylen, Phenylethenyl, Phenylethinyl, Benzyl, Cyclohexyl, Phenoxy, Methoxyphenyl, Formyl oder Phenyl substituiertes und/oder benzanelliertes Phenyl, Naphthyl, Phenoxy, Cyclohexenyl, Cyclohexyl, Benzyl, Pyridyl, Pyrimidyl, Pyrazolyl, Oxazolyl, Thiazolyl, Thienyl, Furyl, Thiadiazolyl, Oxadiazolyl, Imidazolyl oder Triazolyl steht
und als zusätzliche Pyrimidylsubstituenten jeweils infrage kommen: Fluor, Chlor, Brom, Cyano, Nitro, Methyl, Ethyl, Methoxy, Ethoxy, Methylthio, Trifluormethyl, Trichlormethyl, Trifluormethoxy, Trifluormethylthio, Methoxycarbonyl, Ethoxycarbonyl Dimethylamino, Cyclopentyl, Cyclohexyl, 1,3-Propandiyl oder 1,4-Butandiyl und wobei
R⁴ für Methyl, Ethyl oder Benzyl steht.

4. Verfahren zur Herstellung von pyrimidyl-substituierten Acrylsäureestern der allgemeinen Formel (I), 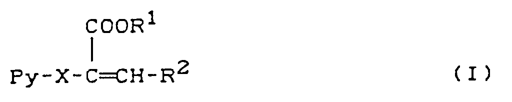 in welcher
R¹, R², X und Py die in Anspruch 1 angegebenen Bedeutungen hat
deren Isomere oder Isomerengemische, dadurch gekennzeichnet, daß man
substituierte Essigsäureester der Formel (II),
Py-X-CH₂-COOR¹ (II)
in welcher
R¹, Py und X die oben angegebene Bedeutung haben,
mit Alkoxy-bis-(dialkylamino)-methan-Verbindungen der Formel (III), 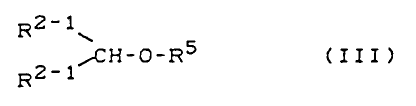 in welcher
R²⁻¹ für Dialkylamino steht und
R⁵ für Alkyl oder Cycloalkyl steht,
gegebenenfalls in Gegenwart eines Verdünnungsmittels umsetzt und gegebenenfalls anschließend in einer 2. Stufe die so erhältlichen 3-Dialkylaminoacrylsäurederivate der Formel (Ia), 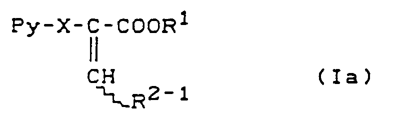 in welcher
R¹, R²⁻¹, Py und X die oben angegebene Bedeutung haben, mit verdünnten Mineralsäuren hydrolysiert und anschließend in einer 3. Stufe die so erhältlichen 3-Hydroxyacrylsäureester der Formel (IV), 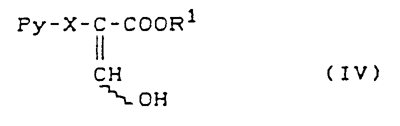 in welcher
R¹, X und Py die oben angegebene Bedeutung haben,
mit Alkylierungsmitteln der Formel (V),
R³-E¹ (V)
in welcher
E¹ für eine elektronenanziehende Abgangsgruppe steht und
R³ die oben angegebene Bedeutung hat,
gegebenenfalls in Gegenwart eines Verdünnungsmittels und gegebenenfalls in Gegenwart eines Reaktionshilfsmittels umsetzt.

5. Schädlingsbekämpfungsmittel, gekennzeichnet durch einen Gehalt an mindestens einem pyrimidyl-substituierten Acrylsäureester der Formel (I) nach den Ansprüchen 1 bis 4.

6. Verwendung von pyrimidyl-substituierten Acrylsäureestern der Formel (I) nach den Ansprüchen 1 bis 4 zur Bekämpfung von Schädlingen.

7. Verfahren zur Bekämpfung von Schädlingen, dadurch gekennzeichnet, daß man pyrimidyl-substituierte Acrylsäureester der Formel (I) nach den Ansprüchen 1 bis 4 auf Schädlinge und/oder ihren Lebensraum einwirken läßt.

8. Verfahren zur Herstellung von Schädlingsbekämpfungsmitteln, dadurch gekennzeichnet, daß man pyrimidylsubstituierte Acrylsäureester der Formel (I) nach den Ansprüchen 1 bis 4 mit Streckmitteln und/oder oberflächenaktiven Mitteln vermischt.

9. 3-Hydroxyacrylsäureester der Formel (IV) 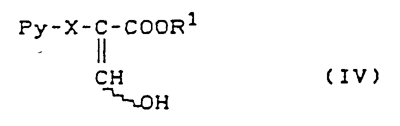 in welcher
Py, X und R¹ die in Anspruch 4 angegebene Bedeutung haben.

## Claims

1. Pyrimidyl-substituted acrylic esters of the general formula (I) 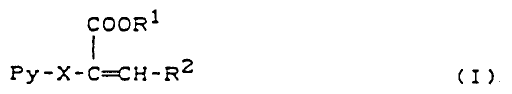 in which
R¹ represents a straight-chain or branched alkyl having 1 to 6 carbon atoms,
R² represents dialkylamino having in each case 1 to 6 carbon atoms in the individual alkyl moieties, or represents a radical -O-R³,
X represents oxygen, sulphur or a radical 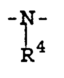 and
Py represents 2-pyrimidyl, 4-pyrimidyl or 5-pyrimidyl, each of which is monosubstituted or polysubstituted by identical or different substituents, suitable substituents in each case being:
halogen, cyano, nitro, in each case straight-chain or branched alkyl, alkoxy or alkylthio, each of which has 1 to 9 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl, alkoximinoalkyl, dialkylamino or dialkylaminocarbonyl, each of which has 1 to 4 carbon atoms in the individual alkyl moieties, in each case straight-chain or branched alkenyl or alkinyl, each of which has 2 to 8 carbon atoms, cycloalkyl or cycloalkenyl, each of which has 3 to 7 carbon atoms and each of which is optionally monosubstituted or polysubstituted by C₁-C₄-alkyl, or represents double-linked alkanediyl having 3 to 5 carbon atoms, or represents aryl, aryloxy, arylthio, arylamino, N-alkyl-arylamino, arylcarbonyl, aralkyl, arylalkenyl, arylalkinyl, arylalkyloxy, arylalkylthio, heteroaryl, heteroaryloxy, heteroarylthio, heteroarylamino, N-alkyl-heteroarylamino, heteroarylcarbonyl, heteroarylalkyl, heteroarylalkenyl, heteroarylalkinyl, heteroarylalkyloxy or heteroarylalkylthio, each of which has 6 to 10 carbon atoms in the aryl moiety or 2 to 9 carbon atoms and 1 to 4 identical or different heteroatoms - in particular nitrogen, oxygen and/or sulphur - in the heteroaryl moiety and, if appropriate, 1 to 6 carbon atoms in the straight-chain or branched alkyl moiety or, if appropriate, 2 to 6 carbon atoms in the straight-chain or branched alkenyl or alkinyl moiety, and each of which is optionally monosubstituted or poly-substituted in the aryl moiety or heteroaryl moiety by identical or different substituents from the series comprising
- halogen, alkyl, cycloalkyl, alkoxy, alkylthio, halogenoalkyl, halogenoalkoxy, halogenoalkylthio, formyl, dioxyalkylene, halogen-substituted dioxyalkylene, or in each case optionally substituted phenyl, phenoxy, benzyl, phenylethyl, phenylethenyl or phenylethinyl;
where
R³ represents a straight-chain or branched alkyl having 1 to 6 carbon atoms or aralkyl having 1 to 4 carbon atoms in the straight-chain or branched alkyl moiety and 6 to 10 carbon atoms in the aryl moiety, and
R⁴ represents hydrogen, straight-chain or branched alkyl having 1 to 6 carbon atoms, or represents aralkyl or aryl each of which has 1 to 6 carbon atoms in the straight-chain or branched alkyl moiety and 6 to 10 carbon atoms in the particular aryl moiety, each of which is optionally monosubstituted or poly-substituted in the aryl moiety by identical or different substituents, suitable substituents in the aryl moiety in each case being those mentioned in the case of Py.

2. Pyrimidyl-substituted acrylic esters of the formula (I) according to Claim 1, in which
R¹ represents methyl, ethyl, n- or i-propyl, or n-, i-, s- or t-butyl,
R² represents dialkylamino having in each case 1 to 4 carbon atoms in the individual alkyl moieties, or represents a radical -O-R³,
X represents oxygen, sulphur or a radical 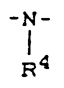 and
Py represents 2-pyrimidyl or 4-pyrimidyl, each of which is monosubstituted to trisubstituted by identical or different substituents, where at least one substituent represents phenyl, naphthyl, phenoxy, phenylthio, N-methyl-phenylamino, phenylcarbonyl, benzyl, phenylethyl, phenylpropyl, phenylethenyl, phenylethinyl, benzyloxy, cyclohexenyl or heteroaryl, each of which is optionally monosubstituted to tri-substituted by identical or different substituents from the series comprising
- fluorine, chlorine, bromine, methyl, ethyl, n- or i-propyl, n-, i-, s- or t-butyl, methoxy, ethoxy, n- or i-propoxy, methylthio, ethylthio, trifluoromethyl, trifluoromethoxy, formyl, trifluoromethylthio, dioxymethylene, dioxyethylene, tetrafluorodioxyethylene, difluorodioxymethylene, cyclopentyl, cyclohexyl, or comprising phenyl, phenoxy, benzyl, phenylethyl, phenylethenyl or phenylethinyl, each of which is optionally monosubstituted to tri-substituted by identical or different substituents [by fluorine, chlorine, methyl, methoxy or trifluoromethyl] -
and suitable individual heteroaryl radicals being those which follow: 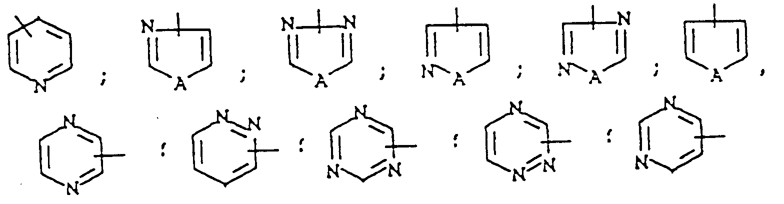 which can also optionally be benzo-fused and in which
A in each case represents oxygen, sulphur or an NH group;
in addition, the following other pyrimidyl substituents are preferably suitable: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trichloromethoxy, trifluoromethylthio, methoxycarbonyl, ethoxycarbonyl, methoximinomethyl, ethoximinomethyl, methoximinoethyl, ethoximinoethyl, dimethylamino, diethylamino, dimethylcarbamoyl, diethylcarbamoyl, allyl, butenyl, ethinyl, vinyl, propargyl, cyclopentyl, cyclohexyl, 1,3-propanediyl or 1,4-butanediyl;
where
R³ represents methyl, ethyl, n- or i-propyl, n-, i-, s- or t-butyl or benzyl and
R⁴ represents hydrogen, methyl, ethyl, n- or i-propyl, n-, i-, s- or t-butyl, or benzyl or phenyl.

3. Pyrimidyl-substituted acrylic esters of the formula (I) according to Claim 1, in which
R¹ represents methyl or ethyl,
R² represents methoxy or ethoxy,
X represents a radical 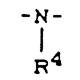 and
Py represents 2-pyrimidyl or 4-pyrimidyl, each of which is monosubstituted to trisubstituted by identical or different substituents, where at least one substituent in each case represents phenyl, naphthyl, phenoxy, cyclohexenyl, cyclohexyl, benzyl, pyridyl, pyrimidyl, pyrazolyl, oxazolyl, thiazolyl, thienyl, furyl, thiadiazolyl, oxadiazolyl, imidazolyl or triazolyl, each of which is optionally benzo-fused and/or monosubstituted to trisubstituted by identical or different substituents from the series comprising fluorine, chlorine, bromine, methyl, ethyl, t-butyl, methoxy, ethoxy, trifluoromethyl, trifluoromethoxy, dioxymethylene, difluorodioxymethylene dioxyethylene, tetrafluorodioxyethylene, phenylethenyl, phenylethinyl, benzyl, cyclohexyl, phenoxy, methoxyphenyl, formyl or phenyl, and the following are suitable in each case as additional pyrimidyl substituents: fluorine, chlorine, bromine, cyano, nitro, methyl, ethyl, methoxy, ethoxy, methylthio, trifluoromethyl, trichloromethyl, trifluoromethoxy, trifluoromethylthio, methoxycarbonyl, ethoxycarbonyl dimethylamino, cyclopentyl, cyclohexyl, 1,3-propanediyl or 1,4-butanediyl, and where
R⁴ represents methyl, ethyl or benzyl.

4. Process for the preparation of pyrimidyl-substituted acrylic esters of the general formula (I) 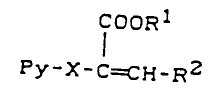 in which R¹, R², X and Py have the meanings given in Claim 1,
and their isomers or mixtures of isomers, characterized in that substituted acetic esters of the formula (II)
Py-X-CH₂-COOR¹ (II) (II)
in which
R¹, Py and X have the abovementioned meaning are reacted with alkoxy-bis-(dialkylamino)-methane compounds of the formula (III) 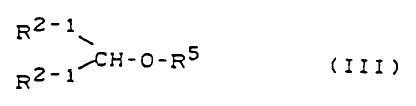 in which
R²⁻¹ represents dialkylamino and
R⁵ represents alkyl or cycloalkyl,
if appropriate in the presence of a diluent and, if appropriate, the resulting 3-dialkylaminoacrylic acid derivatives of the formula (Ia) 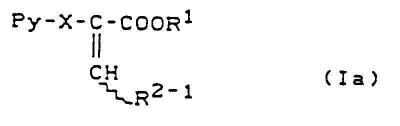 in which
R¹, R²⁻¹, Py and X have the abovementioned meaning,
are, in a 2nd step, subsequently hydrolysed with dilute mineral acids and the resulting 3-hydroxyacrylic esters of the formula (IV) 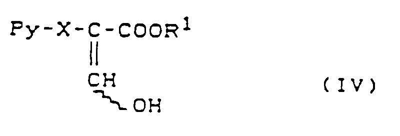 in which
R¹, X and Py have the abovementioned meaning, are, in a 3rd step, subsequently reacted with alkylating agents of the formula (V)
R³-E¹ (V)
in which
E¹ represents an electron-attracting leaving group
and
R³ has the abovementioned meaning,
if appropriate in the presence of a diluent and if appropriate in the presence of a reaction auxiliary.

5. Pesticides, characterised in that they contain at least one pyrimidyl-substituted acrylic ester of the formula (I) according to Claims 1 to 4.

6. Use of pyrimidyl-substituted acrylic esters of the formula (I) according to Claims 1 to 4, for combating pests.

7. Method of combating pests, characterised in that pyrimidyl-substituted acrylic esters of the formula (I) according to Claims 1 to 4 are allowed to act on pests and/or their habitat.

8. Process for the preparation of pesticides, characterised in that pyrimidyl-substituted acrylic esters of the formula (I) according to Claims 1 to 4 are mixed with extenders and/or surface-active agents.

9. 3-hydroxyacrylic esters of the formula (IV) 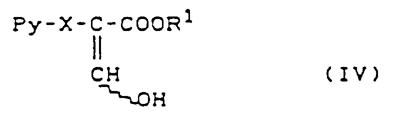 in which
Py, X and R¹ have the meaning given in Claim 4.

## Revendications

1. Esters d'acides acryliques substitués par un groupe pyrimidyle, de formule générale (I) 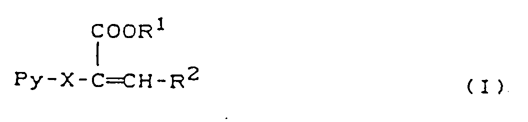 dans laquelle
R₁ est un groupe alkyle linéaire ou ramifié ayant 1 à 6 atomes de carbone,
R² désigne un groupe dialkylamino ayant 1 à 6 atomes de carbone dans chacune des parties alkyle individuelles ou représente un reste -O-R³,
X est de l'oxygène, du soufre ou un reste 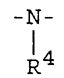 et
Py représente un groupe 2-pyrimidyle, 4-pyrimidyle ou 5-pyrimidyle portant dans chaque cas un ou plusieurs substituants identiques ou différents, les substituants considérés étant les suivants : un halogène, un groupe cyano, nitro, un groupe alkyle, alkoxy ou alkylthio linéaire ou ramifié ayant chacun 1 à 9 atomes de carbone, un groupe halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle, alkoximinoalkyle, dialkylamino ou dialkylaminocarbonyle linéaire ou ramifié ayant chacun 1 à 4 atome de carbone dans les parties alkyle individuelles, un groupe alcényle ou alcynyle linéaire ou ramifié ayant chacun 2 à 8 atomes de carbone, un groupe cycloalkyle ou cycloalcényle ayant chacun 3 à 7 atomes de carbone et portant chacun le cas échéant un ou plusieurs substituants alkyle en C₁ à C₄, un groupe alcanediyle divalent ayant 3 à 5 atomes de carbone ou un groupe aryle, aryloxy, arylthio, arylamino, N-alkylarylamino, arylcarbonyle, aralkyle, arylalcényle, arylalcynyle, arylalkyloxy, arylalkylthio, hétéroaryle, hétéroaryloxy, hétéroarylthio, hétéroarylamino, N-alkylhétéroarylamino, hétéroarylcarbonyle, hétéroarylalkyle, hétéroarylalcényle, hétéroarylalcynyle, hétéroarylalkyloxy ou hétéroarylalkylthio ayant chacun 6 à 10 atomes de carbone dans la partie aryle ou 2 à 9 atomes de carbone et 1 à 4 hétéroatomes identiques ou différents - notamment azote, oxygène et/ou soufre - dans la partie hétéroaryle et le cas échéant 1 à 6 atomes de carbone dans la partie alkyle linéaire ou ramifiée ou le cas échéant 2 à 6 atomes de carbone dans la partie alcényle ou alcynyle linéaire ou ramifié, chacun d'eux étant éventuellement substitué une ou plusieurs fois, identiques ou différentes, dans la partie aryle ou dans la partie hétéroaryle par
- un halogène, un radical alkyle, cycloalkyle, alkoxy, alkylthio, halogénalkyle, halogénalkoxy, halogénalkylthio, fomyle, dioxyalkylène, dioxyalkylène substitué par un halogène ou phényle, phénoxy, benzyle, phényléthyle, phényléthényle ou phényléthinyle éventuellement substitué ;
R³ représente un groupe alkyle linéaire ou ramifié ayant 1 à 6 atomes de carbone ou un groupe aralkyle ayant 1 à 4 atomes de carbone dans la partie alkyle linéaire ou ramifiée et 6 à 10 atomes de carbone dans la partie aryle, et
R⁴ représente de l'hydrogène, un groupe alkyle linéaire ou ramifié ayant 1 à 6 atomes de carbone, ou un groupe aralkyle ou aryle ayant 1 à 6 atomes de carbone dans la partie alkyle linéaire ou ramifié et 6 à 10 atomes de carbone dans chaque partie aryle, chacun étant éventuellement substitué une ou plusieurs fois identiques ou différentes dans la partie aryle, avec comme substituants dans la partie aryle les substituants mentionnés dans le cas de Py.

2. Esters d'acides acryliques substitués par un groupe pyrimidyle, de formule (I) suivant la revendication 1, dans lesquels
R¹ est un groupe méthyle, éthyle, n-propyle, isopropyle, n-butyle, isobutyle, sec.-butyle ou tertio-butyle,
R² est un groupe dialkylamino ayant 1 à 4 atomes de carbone dans chacune des parties alkyle, ou un reste -O-R³,
X représente l'oxygène, le soufre, ou un reste 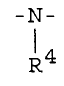 et
Py est un groupe 2-pyrimidyle, 4-pyrimidyle portant chacun un à trois substituants identiques ou différents, l'un au moins d'un substituants représentant dans chaque cas un groupe phényle, naphtyle, phénoxy, phénylthio, N-méthylphénylamino, phénylcarbonyle, benzyle, phényléthyle, phénylpropyle, phényléthényle, phényléthinyle, benzyloxy, cyclohexényle ou hétéroaryle substitué chacun le cas échéant une à trois fois identiques ou différentes par
- du fluor, du chlore, du brome, un radical méthyle, éthyle, n-propyle, isopropyle, n-butyle, isobutyle, sec.-butyle, tertio-butyle, méthoxy, éthoxy, n-propoxy, isopropoxy, méthylthio, éthylthio, trifluorométhyle, trifluorométhoxy, formyle, trifluorométhylthio, dioxyméthylène, dioxyéthylène, tétrafluorodioxyéthylène, difluorodioxyméthylène, cyclopentyle, cyclohexyle, ou un radical phényle, phénoxy, benzyle, phényléthyle, phényléthényle, ou phényléthinyle portant le cas échéant un à trois substituants, identiques ou différents, fluoro, chloro, méthyle, méthoxy ou trifluorométhyle, les restes hétéroaryle considérés étant en particulier les restes suivants : 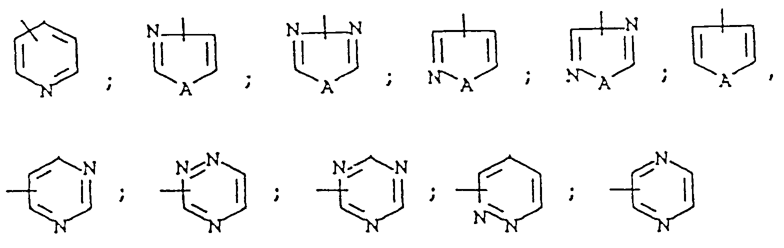 qui peuvent aussi être le cas échéant condensés au benzène et dans lesquels
A représente dans chaque cas de l'oxygène, du soufre ou un groupe NH ;
en considérant en outre comme autre substituant du groupe pyrimidyle de préférence les substituants suivants :
fluor, chlor, brome, cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, trifluorométhyle, trifluorométhoxy, trichlorométhoxy, trifluorométhylthio, méthoxycarbonyle, éthoxycarbonyle, méthoximinométhyle, éthoximinométhyle, méthoximinoéthyle, éthoximinoéthyle, diméthylamino, diéthylamino, diméthylcarbamoyle, diéthylcarbamoyle, allyle, butényle, éthinyle, vinyle, propargyle, cyclopentyle, cyclohexyle, 1,3-propanediyle ou 1,4-butanediyle ;
R³ est un groupe méthyle, éthyle, n-propyle, isopropyle, n-butyle, isobutyle, sec.-butyle, tertio-butyle ou benzyle et
R⁴ est de l'hydrogène, un groupe méthyle, éthyle, n-propyle, isopropyle, n-butyle, isobutyle, sec.-butyle, tertio-butyle ou un groupe benzyle ou phényle.

3. Esters d'acides acryliques substitués par un groupe pyrimidyle, de formule (I) suivant la revendication 1, dans lesquels
R¹ est un groupe méthyle ou éthyle,
R² est un groupe méthoxy ou éthoxy,
X est un reste 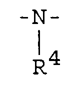 et
Py représente un groupe 2-pyrimidyle ou 4-pyrimidyle portant chacun le cas échéant un à trois substituants identiques ou différents, l'un au moins des substituants étant dans chaque cas un groupe phényle, napthyle, phénoxy, cyclohexényle, cyclohexyle, benzyle, pyridyle, pyrimidyle, pyrazolyle, oxazolyle, thiazolyle, thiényle, furyle, thiadiazolyle, oxadiazolyle, imidazolyle, ou triazolyle portant chacun le cas échéant un à trois substituants, identiques ou différents, fluor, chlore, brome, méthyle, éthyle, tertio-butyle, méthoxy, éthoxy, trifluorométhyle, trifluorométhoxy, dioxyméthylène, difluorodioxyméthylène, dioxyéthylène, tétrafluorodioxyéthylène, phényléthényle, phényléthinyle, benzyle, cyclohexyle, phénoxy, méthoxyphényle, formyle ou phényle et/ou chacun étant éventuellement condensé à du benzène, et on considère comme autres substituants du groupe pyrimidyle : le fluor, le chlore, le brome, un radical cyano, nitro, méthyle, éthyle, méthoxy, éthoxy, méthylthio, trifluorométhyle, trichlorométhyle, trifluorométhoxy, trifluorométhylthio, méthoxycarbonyle, éthoxycarbonyle, diméthylamino, cyclopentyle, cyclohexyle, 1,3-propanediyle ou 1,4-butanediyle et
R⁴ est un groupe méthyle, éthyle ou benzyle.

4. Procédé de production d'esters d'acides acryliques substitués par un groupe pyrimidyle, de formule générale (I) 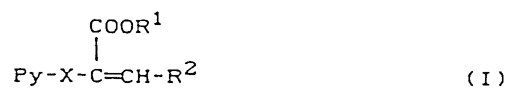 dans laquelle
R¹, R², X et Py ont les définitions indiquées dans la revendication 1,
de leurs isomères ou de leurs mélanges d'isomères, caractérisé en ce qu'on fait réagir des esters d'acides acétiques substitués de formule (II)
Py-X-CH₂-COOR¹ (II)
dans laquelle
R¹, Py et X ont la définition indiquée ci-dessus, avec des alkoxy-bis-(dialkylamino)-méthanes de formule (III) 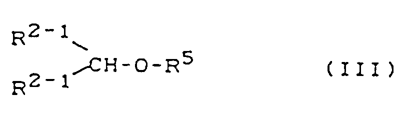 dans laquelle
R²⁻¹ est un groupe dialkylamino et
R⁵ est un groupe alkyle ou cycloalkyle,
le cas échéant en présence d'un diluant, après quoi on hydrolyse le cas échéant dans une deuxième étape les dérivés d'acides 3-dialkylaminoacryliques ainsi obtenus de formule (Ia) 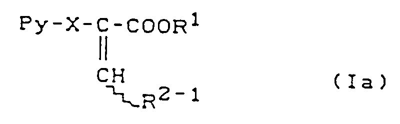 dans laquelle
R¹, R²⁻¹, Py et X ont la définition indiquée ci-dessus, avec des acides minéraux dilués puis on fait réagir dans une troisième étape les esters d'acides 3-hydroxyacryliques ainsi obtenus de formule (IV) 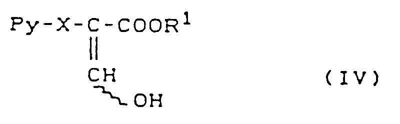 dans laquelle
R¹, X et Py ont la définition indiquée ci-dessus, avec des agents d'alkylation de formule (V)
R³-E¹ (V)
dans laquelle
E¹ est un groupe partant attirant les électrons et
R³ a la définition indiquée ci-dessus, le cas échéant en présence d'un diluant et en la présence éventuelle d'une substance auxiliaire de réaction.

5. Composition pesticide, caractérisée par une teneur en au moins un ester d'acide acrylique substitué par un groupe pyrimidyle de formule (I) suivant les revendications 1 à 4.

6. Utilisation d'esters d'acides acryliques substitués par un groupe pyrimidyle de formule (I) suivant les revendications 1 à 4 pour combattre des parasites.

7. Procédé pour combattre des parasites, caractérisé en ce qu'on fait agir des esters d'acides acryliques substitués par un groupe pyrimidyle de formule (I) suivant les revendications 1 à 4 sur les parasites et/ou sur leur milieu.

8. Procédé de préparation de compositions pesticides, caractérisé en ce qu'on mélange des esters d'acides acryliques substitués par un groupe pyrimidyle de formule (I) suivant les revendications 1 à 4 avec des diluants et/ou des agents tensio-actifs.

9. Esters d'acides 3-hydroxyacryliques de formule (IV) 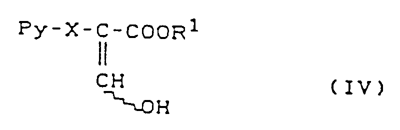 dans laquelle
Py, X et R¹ ont la définition donnée dans la revendication 4.
